(19) Europäisches Patentamt
European Patent Office
Office européen des brevets

(11) **EP 1 212 085 B1**

(12) **EUROPEAN PATENT SPECIFICATION**

(45) Date of publication and mention
of the grant of the patent:
**31.10.2007   Bulletin 2007/44**

(51) Int Cl.:
*A61K 39/39* (2006.01)     *A61K 31/7088* (2006.01)
*A61K 9/127* (2006.01)     *A61P 37/04* (2006.01)
*A61K 35/00* (2006.01)

(21) Application number: **00956004.6**

(22) Date of filing: **28.08.2000**

(86) International application number:
**PCT/CA2000/001013**

(87) International publication number:
**WO 2001/015726 (08.03.2001 Gazette 2001/10)**

(54) **COMPOSITIONS FOR STIMULATING CYTOKINE SECRETION AND INDUCING AN IMMUNE RESPONSE**

ZUSAMMENSETZUNGEN ZUR STIMULATION DER ZYTOKIN SEKRETION UND ZUR INDUKTION EINER IMMUNANTWORT

COMPOSITIONS STIMULANT LA SECRETION DE CYTOKINE ET PROVOQUANT UNE REACTION IMMUNITAIRE

(84) Designated Contracting States:
**AT BE CH CY DE DK ES FI FR GB GR IE IT LI LU MC NL PT SE**

(30) Priority: **27.08.1999   US 151211 P**
**13.01.2000   US 176406 P**

(43) Date of publication of application:
**12.06.2002   Bulletin 2002/24**

(73) Proprietor: **INEX Pharmaceuticals Corp.**
**Burnaby, British Columbia V5J 5J8 (CA)**

(72) Inventors:
• **SEMPLE, Sean, C.**
**Vancouver, British Columbia V6H 2K3 (CA)**
• **HARASYM, Troy, O.**
**North Vancouver, BC, V7K 2K8 (CA)**
• **KLIMUK, Sandra, K.**
**North Vancouver, British Columbia V7N 3N (CA)**
• **KOJIC, Ljiljiana, D.**
**Vancouver, British Columbia V5Z IC9 (CA)**
• **BRAMSON, Jonathan, L.**
**Oakville, Ontario L6H 5C6 (CA)**
• **MUI, Barbara**
**Vancouver, British Columbia V6R 4R2 (CA)**
• **HOPE, Michael, J.**
**Vancouver, British Columbia V6R 2K2 (CA)**

(74) Representative: **Frei, Alexandra Sarah**
**Frei Patentanwaltsbüro**
**Postfach 1771**
**8032 Zürich (CH)**

(56) References cited:
**WO-A-96/02555     WO-A-98/18810**
**WO-A-98/40100     WO-A-98/51278**
**WO-A-98/55495**

• **MAURER NORBERT ET AL: "Lipid-based systems for the intracellular delivery of genetic drugs." MOLECULAR MEMBRANE BIOLOGY, vol. 16, no. 1, January 1999 (1999-01), pages 129-140, XP000982197 ISSN: 0968-7688**
• **MEYER OLIVIER ET AL: "Cationic liposomes coated with polyethylene glycol as carriers for oligonucleotides." JOURNAL OF BIOLOGICAL CHEMISTRY, vol. 273, no. 25, 19 June 1998 (1998-06-19), pages 15621-15627, XP002160291 ISSN: 0021-9258**
• **BOGGS R T ET AL: "CHARACTERIZATION AND MODULATION OF IMMUNE STIMULATION BY MODIFIED OLIGONUCLEOTIDES" ANTISENSE & NUCLEIC ACID DRUG DEVELOPMENT, US, MARY ANN LIEBERT, INC., NEW YORK, vol. 7, 1 October 1997 (1997-10-01), pages 461-471, XP002053418 ISSN: 1087-2906 cited in the application**

- CHU R S ET AL: "CPG OLIGODEOXYNUCLEOTIDES ACT AS ADJUVANTS THAT SWITCH ON T HELPER 1 (TH1) IMMUNITY" JOURNAL OF EXPERIMENTAL MEDICINE,JP,TOKYO, vol. 186, no. 10, 1 November 1997 (1997-11-01), pages 1623-1631, XP002910130 ISSN: 0022-1007
- BRAMSON JONATHAN L ET AL: "Intravenous administration of stabilized antisense lipid particles (SALP) leads to activation and expansion of liver natural killer cells." ANTISENSE & NUCLEIC ACID DRUG DEVELOPMENT, vol. 10, no. 3, June 2000 (2000-06), pages 217-224, XP002160292 ISSN: 1087-2906
- WHITMORE M ET AL: "LPD lipopolyplex initiates a potent cytokine response and inhibits tumor growth." GENE THERAPY, vol. 6, no. 11, November 1999 (1999-11), pages 1867-1875, XP000982147 ISSN: 0969-7128 cited in the application

SEQUENCE LISTING

< 110 > Inex Pharmaceuticals Corp.
Semple, Sean
Harasym, Troy
Klimuk, Sandra
Kojic, Ljiljiana
Bramson, Jonathan
Mui, Barbara

Hope, Michael

< 120> COMPOSITIONS FOR STIMULATING CYTOKINE SECRETION AND INDUCING AN IMMUNE RESPONSE

<130> INEXP006WO

< 150> 60/176,406

< 151 > 2000-01-13

< 150> 60/151,211

< 151 > 1999-08-27

< 160> 11

<170> PatentIn version 3.0

<210> 1

<211> 20

<212> DNA

<213> human

< 220 >

< 221 > 3' untranslated region of human ICAM-1 mRNA

<222> (1) .. (20)

< 400 > 1
gcccaagctg gcatccgtca        20

<210> 2

<211 > 20

<212> DNA

<213> murine

<220>

<221> 3' untranslated region of murine ICAM-1 mRNA

<222> (1)..(20)

<400> 2
tgcatccccc aggccaccat        20

<210> 3

<211> 15

<212> DNA

<213> human

<220>

<221> human epidermal growth factor mRNA, receptor translation termination codon region

<222> (1)..(15)

<400> 3
ccgtggtcat gctcc          15

<210> 4

<211> 16

<212> DNA

<213> human/mouse

<220>

<221> initiation codon region of human/mouse c-myc proto-oncogene mRNA

<222> (1)..(16)

<400> 4
taacgttgag gggcat          16

<210> 5

<211> 15

<212> DNA

<213> human/mouse

<220>

<221 > initiation codon region of human/mouse c-myc proto-oncogene mRNA

<222> (1)..(15)

<400> 5
aacgttgagg ggcat          15

<210> 6

<211> 16

<212> DNA

<213> plasmid

<220>

<221> non-ISS control

<222> (1)..(16)

<400> 6
taagcatacg gggtgt     16

<210> 7

<211> 15

<212> DNA

<213> plasmid

<220>

<221> ISS control

<222> (1)..(15)

<400> 7
aacgagttgg ggcat     15

<210> 8

<211> 24

<212> DNA

<213> plasmid

<220>

<221> hybridizes to c-myb mRNA

<222> (1)..(24)

<400> 8
tatgctgtgc cggggtcttc gggc     24

<210> 9

<211> 18

<212> DNA

<213> plasmid

<220>

<221> hybridizes to IGF-1R mRNA

<222> (1)..(18)

<400> 9
ggaccctcct ccggagcc     18

<210> 10

<211> 15

<212> DNA

<213> plasmid

<220>

<221> control PO

<222> (1)..(15)

<400> 10
aagcatacgg ggtgt        15

<210> 11

<211> 20

<212> DNA

<213> plasmid

<220>

<221> control containing 3 CpG motifs

<222> (1)..(20)

<400> 11
tcgcatcgac ccgcccacta        20

## Description

Background of the Invention

The invention described herein relates to compositions of lipid formulated nucleic acids and their methods of use for inducing an immune response in a mammal. Certain of the compositions employ additional components such as antigens, additional therapeutic agents, and/or other components, but these additional components are not necessary for all applications.

Since the mid-1980's it has been known that nucleic acids, like other macromolecules, can act as biological response modifiers and induce immune responses in mammals upon *in vivo* administration. (Tokunaga et al., 1984; Shimada et al., 1985; Mashiba et al., 1988; Yamamoto et al., 1988; Phipps et al. 1988). Several publications in the early 1990's established that stimulation of an immune response was dependent on the features of the nucleic acid employed. Important features include presence of secondary structure palindromes (Yamamoto 1992a) and the chemistry of the nucleic acid (i.e. methylation status of C nucleotides - dependent on bacterial or mammalian source of DNA (Messina et al. 1991; Yamamoto 1992a) or internucleotide linkage chemistry such as phosphorothioates (Pisetsky and Reich 1993)); as well as nucleotide sequence specific effects, such as poly dG and CpG motifs (Tokunaga et al. 1992; Yamamoto et al 1992b; McIntyre, KW et al. 1993; Pisetsky and Reich, 1993; Yamamoto et al. 1994; Krieg et al. 1995).

The mechanism of action of these immune stimulatory sequences (also in the art called immunostimulatory sequences or "ISS") is suggested to be different from "antisense" or "gene expression" mechanisms which are well known in the art. This results in significantly different potential uses for nucleic acids. A variety of such potential uses are set out by Pisetsky DS. 1996, and others are known in the art. These include use of free-form ISS as immune adjuvants, as vaccines in combination with a variety of antigens (see PCT publication WO 98/40100 to Davis, HL et al. ), and in combination with other bioactive agents. Methods of avoiding immune stimulating effects have also been proposed.

It is highly desirable to further exploit the discovery of ISS and to generate therapeutic products employing them. It is an object of this invention to provide compositions of lipid formulated nucleic acids and their methods of use for inducing an immune response in a mammal. It is also an object to provide lipid-nucleic acid compositions which employ additional components such as antigens, additional therapeutic agents, and/or other components, and their methods of use.

Compositions containing nucleic acids in lipid carriers are known in the art. For example, International Patent Publication No. WO 98/51278 describes lipid-antisense nucleic acid compositions in which lipid mixture including a protonatable lipid and an aggregation-limiting lipid to produce particles in which the nucleic acid is fully encapsulated. These compositions were shown to be therapeutically effective, for example for reduction in tumor size, when antisense nucleic acid complementary to coding nucleic acid sequences in target cells were used.

Summary of the Invention

It has now been surprisingly found that lipid-nucleic acid particles can provide therapeutic benefits, even when the nucleic acid is not complementary to coding sequences in target cells. Thus, it has been found that lipid-nucleic acid particles, including those containing non-sequence specific oligodeoxynucleotides, can be used to stimulate cytokine secretion, thus enhancing the overall immune response of a treated mammal. Further, immune response to specific target antigens can be induced by administration of a antigenic molecule in association with lipid particles containing non-sequence specific oligodeoxynucleotides.

In accordance with the present invention, a method is provided in which therapeutic benefits are provided to a mammal, including a human, by preparing a lipid-nucleic acid particle comprising a nucleic acid which is fully encapsulated in a lipid formulation, which lipid formulation comprises a cationic lipid; and administering the lipid-nucleic acid particle to a mammal. In one embodiment of the invention, the nucleic acid included in lipid-nucleic acid particle is one which may not bind with sequence specificity to particular cells, but which nonetheless, when administered in the combination with the lipid particle is effective to stimulate secretion of cytokines. In a second embodiment of the invention, an antigenic molecule combined with the lipid-nucleic acid particle to induce an immune response specific to a target antigen.

The nucleic acid which is included in the lipid-nucleic acid particle can be a phosphodiester (i.e., an oligodeoxynucleotide consisting of nucleotide residues joined by phosphodiester linkages) or a modified nucleic acid which includes phosphorothioate or other modified linkages, and may suitably be one which is non-complementary to the human genome, such that it acts to provide immunostimulation in a manner which is independent of conventional base-pairing interactions between the nucleic acid and nucleic acids of the treated mammal. In particular, the nucleic acid may suitably contain an immune-stimulating motif such as a CpG motif, or an immune stimulating palindromic sequence.

The cationic lipid included in the nucleic acid particles may be suitably selected from among DODAP, DODMA, DMDMA, DOTAP, DC-Chol, DDAB, DODAC, DMRIE, DOSPA and DOGS. In addition, the lipid particle may suitably contain an modified aggregation-limiting lipid such as a PEG-lipid, a PAO-lipid or a ganglioside.

Brief Description of the Drawings

Figures 1A-C show circulation levels of PEG-liposomes on repeat administration in immune competent Balb/c mice (A), and immune compromised Balb/c nude (B) and Balb/c SCID-Rag2 mice (C).

Figures 2 A and B show influence of nucleic acid sequence (A) and structure (B) on elimination of SALP (PEG-CerC$_{20}$).

Figure 3 shows the influence of the DNA to lipid ratio on liposome recovery.

Figure 4 shows the influence of administration schedule on the onset of the rapid elimination response.

Figures 5 A and B illustrate the role of PEG-lipid in the rapid elimination of liposomes containing ODN.

Figure 6 shows the results of cross-over studies.

Figures 7A and B shows accumulation of AS4200 in Solid Tumors.

Figures 8A and 8B illustrate the enhanced Potency of c-myc/TCS over free c-myc and the influence of Antisense/Lipid ratio.

Figure 9 shows that encapsulated phosphodiester ODN (INX-6298) demonstrates improved efficacy in Murine B16 Melanoma compared to free INX-6298.

Figure 10 shows efficacy of 15mer INX-6298 in DoHH2 human lymphoma in SCID-Rag2 mice.

Figure 11A and 11B illustrate the dose response to free and AS4200 INX-6295 in i.v. DoHH2.

Fig. 12 shows variations in spleen weight in mice treated with various c-myc and lipid formulations.

Figure 13 shows the mitogenicity of various ODN in *in vitro* splenocyte proliferation assay.

Figure 14 shows that mitogenic control ODN INX-4420 demonstrates activity in subcutaneous B 16 Melanoma *in vivo,* similar to LR-3280.

Figure 15 shows that of co-encapsulation of c-myc and conventional drug (doxorubicin) in a single liposome inhibits tumour growth.

Figure 16 illustrates the immunogenicity of AS4204, and the reversal using co-encapsulated doxorubicin.

Figure 17 shows mitogenicity of INX-6295 and INX-6300

Figures 18A and B show increase in mononuclear cells and natural killer activity in the liver following repeated administration of INXC-6295.

Figures 19 A and B show increase in NK1.1+/TCR- cells in the liver following INX-6295/SALP treatment.

Figure 20 shows lack of cytolytic activity in the HMNC from beige mice following INX-6295/SALP treatment.

Figure 21 shows the increase in HMNC following administration of free and encapsulated PS ODN.

Figures 22 A and B shows the increase in NK1.1+/TCR- cells in the liver following SALP treatment.

Figures 23A-C show activation of Natural Killer cells within the HMNC population following administration of free and encapsulated PS ODN.

Figures 24A-C shows transfection profiles of lipoplexes containing either DODAC, DOTAP or DOTMA.

Figure 25 shows the level of cellular infiltrate in the peritoneum following lipoplex administration.

Figures 26A-C show lipoplex induced inflammation is associated with increased production of IFN-$\gamma$.

Figure 27 shows lipoplex induced activation ofNK cells.

Figures 28A-D show serum cytokines indiced by free and liposomal c-myc PS ODN.

Figures 29A-D show serum cytokines indiced by free and liposomal c-myc PS ODN.

Figures 30A-D show results of a test comparing cytokine secretion by PO and PS ODN.

Figures 31A and B show the two phases of IFN-( induction.

Figures 32 A and B show levels of serum IL-12 at a time corresponding to the second phase of IFN-( induction.

Figure 33 shows the effect of ODN dose on serum cytokine induction.

Detailed Description of the Invention

In its broadest sense, the invention described herein relates to compositions of lipid formulated nucleic acids and their methods of use for stimulating cytokine secretion and inducing an immune response to a target antigen in a mammal. Certain of the compositions employ additional components such as antigens, additional therapeutic agents, and/or other components, but these additional components are not necessary for all applications.

As used in the specification and claims hereof, the term "stimulating cytokine secretion" refers to an increase in the amount of one or more cytokines secreted by an organism to whom the compositions of the invention are administered as a proximal result of such administration.

As used in the specification and claims of this application, the term "inducing an immune response" refers to either the generation of an initial immune response or the enhancement of a preexisting immune response to a target antigen.

A. Lipid-Nucleic Acid Compositions

A.1 Nucleic acids

Each of the compositions of the invention includes a nucleic acid. Any nucleic acid may be used, but commonly employed are large double stranded plasmid DNA (500 - 50,000 bp) or short, single stranded oligonucleotides (sometimes called ODN or oligodeoxynucleotides) of 8 - 50 nt. The standard nucleic acid includes phosphodiester linkages between nucleotides, but these linkages may be of any chemistry including phosphorothioate, phosphoramidate, etc. Numerous other chemical modifications to the base, sugar or linkage moieties are also useful. Bases may be methylated or unmethylated. Preferred nucleic acid chemistries are poly-anionic to co-operate with the preferred manufacturing processes described below. Nucleotide sequences may be complementary to patient/subject mRNA, such as antisense oligonucleotides, or they may be foreign or non-complementary (which means they do not specifically hybridize to the patient/subject genome). Sequences may be expressible, such as gene sequences linked to appropriate promoters and expression elements, generally as part of a larger plasmid construct. The sequences may be immune-stimulatory sequences ("ISS"), such as certain palindromes leading to hairpin secondary structures (see Yamamoto S., et al. (1992) J. Immunol. 148: 4072-4076), or CpG motifs (see below), or other known ISS features (such as multi-G domains, see WO 96/11266); or they may be non-ISS sequences, or they may be immune neutralizing motifs which suppress the activity of CpG motifs. Many ISS, non-ISS and neutralizing motifs are well known in the art.

ISS known as CpG motifs are unmethylated cytidine-guanosine dinucleotides within a specific pattern of flanking bases (Kreig, A.M. et al. (1995) Nature 374, 546-549). See also PCT Publication No. WO 96/02555; PCT Publication No. WO 98/18810; PCT Publication No. WO 98/40100; U.S. Patent No. 5,663,153; U.S. Patent No. 5,723,335. The base context of CpG motifs is clearly crucial for ISS activity, since many CpG motifs are not immune stimulatory. The most dramatic effects on the immune stimulatory properties of a particular DNA sequence generally come from changes to the two bases immediately flanking the CpG dinucleotide (on the 5' and 3' sides). Even single changes can convert an ISS motif to a non-ISS motif. Further, back to back CpG dinucleotides, CCG trinucleotides or CGG trinucleotides, alone or in combination, could be neutralizing motifs that block the immune stimulatory effects of CpG motifs. (Kreig, AM (1999) J Gene Med 1: 56-63).

ISS, non-ISS and neutralizing motif sequences may be organism specific. The immune stimulating capacity of a sequence in an organism can be determined by simple experimentation comparing the sequence in question with other adjuvants, or by measuring activation of host defense mechanisms, induction of immune system components, etc., all as well known in the art. A non-ISS sequence does not stimulate the immune system or induce an immune response when administered, in free form, to a naive mammal.

A.2 Lipids and other components of particles

Besides nucleic acids, the compositions of the invention employ lipids and may employ other components.

The term "lipid" refers to a group of organic compounds that are esters of fatty acids and are characterized by being insoluble in water but soluble in many organic solvents. They are usually divided in at least three classes: (1) "simple lipids" which include fats and oils as well as waxes; (2) "compound lipids" which include phospholipids and glycolipids; and (3) "derived lipids" such as steroids and compounds derived from lipid manipulations. A wide variety of lipids may be used with the invention, some of which are described below.

The term "charged lipid" refers to a lipid species having either a cationic charge or negative charge or which is a zwitterion which is not net neutrally charged, and generally requires reference to the pH of the solution in which the lipid is found.

Cationic charged lipids at physiological pH include, but are not limited to, N,N-dioleyl-N,N-dimethylammonium chloride ("DODAC"); N-(2,3-dioleyloxy)propyl)-N,N,N-trimethylammonium chloride ("DOTMA"); N,N-distearyl-N,N-dimethylammonium bromide ("DDAB"); N-(2,3-dioleyloxy)propyl)-N,N,N-trimethylammonium chloride ("DOTAP"); 3β-(N-(N',N'-dimethylaminoethane)-carbamoyl)cholesterol ("DC-Chol") and N-(1,2-dimyristyloxyprop-3-yl)-N,N-dimethyl-N-hydroxyethyl ammonium bromide ("DMRIE"). Additionally, a number of commercial preparations of catioinic lipids are available which can be used in the present invention. These include, for example, Lipofectin™ (commercially available cationic liposomes comprising DOTMA and 1,2-dioleoyl-sn-3-phosphoethanolamine ("DOPE"), from GIBCO/BRL, Grand Island, New York, USA); Lipofectamine™ (commercially available cationic liposomes comprising N-(1-(2,3-dioleyloxy) propyl)-N-(2-(sperminecarboxamido)ethyl)-N,N-dimethylammonium trifluoroacetate ("DOSPA") and DOPE from GIBCO/BRL); and Transfectam™ (commercially available cationic lipids comprising dioctadecylamidoglycyl carboxyspermine ("DOGS") in ethanol from Promega Corp., Madison, Wisconsin, USA).

Some cationic charged lipids are titrateable, that is to say they have a pKa at or near physiological pH, with the significant consequence for this invention that they are strongly cationic in mild acid conditions and weakly (or not) cationic at physiological pH. Such cationic charged lipids include, but are not limited to, N-(2,3-dioleyloxy)propyl)-N,N-dimethylammonium chloride ("DODMA") and 1,2-Dioleoyl-3-dimethylammonium-propane ("DODAP"). DMDMA is also a useful titrateable cationic lipid.

Anionic charged lipids at physiological pH include, but are not limited to, phosphatidyl inositol, phosphatidyl serine, phosphatidyl glycerol, phosphatidic acid, diphosphatidyl glycerol, poly(ethylene glycol)-phosphatidyl ethanolamine, dimyristoylphosphatidyl glycerol, dioleoylphosphatidyl glycerol, dilauryloylphosphatidyl glycerol, dipalmitoylphosphatidyl glycerol, distearyloylphosphatidyl glycerol, dimyristoyl phosphatic acid, dipalmitoyl phosphatic acid, dimyristoyl phosphatidyl serine, dipalmitoyl phosphatidyl serine, brain phosphatidyl serine, and the like.

Some anionic charged lipids may be titrateable, that is to say they would have a pKa at or near physiological pH, with the significant consequence for this invention that they are strongly anionic in mild base conditions and weakly (or not) anionic at physiological pH. Such anionic charged lipids can be identified by one skilled in the art based on the principles disclosed herein.

The term "neutral lipid" refers to any of a number of lipid species which exist either in an uncharged or neutral zwitterionic form a physiological pH. Such lipids include, for example, diacylphosphatidylcholine, diacylphosphatidylethanolamine, ceramide, sphingomyelin, cephalin, cholesterol, cerebrosides and diacylglycerols.

Certain preferred lipid formulations used in the invention include aggregation preventing compounds such as PEG-lipids or polyamide oligomer-lipids (such as an ATTA-lipid), and other steric-barrier or "stealth"-lipids, detergents, and the like. Such lipids are described in US Patent Nos. 4320121 to Sears, 5,820,873 to Choi et al., 5,885,613 to Holland et al., WO 98/51278 (inventors Semple et al.), and US Patent Application Serial No. 09/218988 relating to polyamide oligomers, all incorporated herein by reference. These lipids and detergent compounds prevent precipitation and aggregation of formulations containing oppositely charged lipids and therapeutic agents. These lipids may also be employed to improve circulation lifetime *in vivo* (see Klibanov et al. (1990) FEBS Letters, 268 (1): 235-237), or they may be selected to rapidly exchange out of the formulation *in vivo* (see US Pat. No. 5885613).

A preferred embodiment of the invention employs exchangeable steric-barrier lipids (as described in US Patents No. 5,820,873, 5,885,613, and US Pat. Applic. S.N. 09/094540 and 09/218988, assigned to the assignee of the instant invention and incorporated herein by reference). Exchangeable steric-barrier lipids such as $PEG_{2000}$-CerC 14 and ATTA8-CerC 14 are steric-barrier lipids which rapidly exchange out of the outer monolayer of a lipid particle upon administration to a subject/patient. Each such lipid has a characteristic rate at which it will exchange out of a particle depending on a variety of factors including acyl chain length, saturation, size of steric barrier moiety, membrane composition and serum composition, etc. Such lipids are useful in preventing aggregation during particle formation, and their accelerated departure from the particle upon administration provides benefits, such as programmable fusogenicity and particle destabilizing activity, as described in the above noted patent submissions.

Some lipid particle formulations may employ targeting moieties designed to encourage localization of liposomes at certain target cells or target tissues. Targeting moieties may be associated with the outer bilayer of the lipid particle (i.e. by direct conjugation, hydrophobic interaction or otherwise) during formulation or post-formulation. These methods are well known in the art. In addition, some lipid particle formulations may employ fusogenic polymers such as PEAA, hemagluttinin, other lipo-peptides (see US Patent applications SN 08/835,281, and 60/083,294, all incorporated herein by reference) and other features useful for *in vivo* and/or intracellular delivery.

A.3 Other Drug Components

Some preferred embodiments of the invention further comprise other drugs or bioactive agents. These additional components may provide direct additional therapeutic benefit or additional immune-stimulating benefits. In the examples below, doxorubicin (hydroxydaunorubicin), a well know chemotherapeutic agent, is co-encapsulated with the nucleic acid in particles of the invention. Other drugs or bioactive agents may similarly be employed depending on desired application of the invention. Cytotoxic agents include all compounds with cell killing ability, including without limitation cyclophosphamide, dicarbazine, taxanes, camptothecins, vincristine and other vinca alkaloids, cisplatin. Another specific examples is RITUXIN™ (Rituximab) for treatment of Non-Hodgkin's Lymphoma. Anti-bacterial agents such as ciprofloxacin can be useful. , In short, all bioactive agents known in the art which can be incorporated into lipid particles are potential candidates for additional components.

In one embodiment of the invention, the drugs or other bioactive agents are suitably provided in association with the lipid-nucleic acid particle. As used in the specification and claims of this application, the term "in association" refers to co-encapsulation of the drug or bioactive agent with the nucleic acid within the lumen or intralamellar spaces of a lipid particle, disposed within or partially within the lipid membrane, or bonded (covalently or ionically) to the exterior of the lipid particle.

As an alternative to association of drugs or bioactive agents with the lipid particle, the compositions of the invention may include the drugs or bioactive agents that are not associated with the lipid-nucleic acid particle. Such drugs or bioactive agents may be in separate lipid carriers. For example, liposomal vincristine (OncoTCS™) may be used.

A.4 Vaccine components

The invention herein demonstrates that compositions of the invention raise a strong humoral response to PEG-lipid, a normally non-immunogenic or slightly immunogenic compound. Certain embodiments of the invention employ other antigen molecules as part of vaccine compositions. The antigen molecules may be antigens which are inhernetly immunogenic, or they may be non-immunogenic or slightly immunogenic antigens. These antigens include foreign or homologous antigens and include HBA - hepatitis B antigen (recombinant or otherwise); other hepatitis peptides; HIV proteins GP120 and GP160; Mycoplasma cell wall lipids; any tumour associated antigen; Carcinoembryonic Antigen (CEA); other embryonic peptides expressed as tumor specific antigens; bacterial cell wall glycolipids; Gangliosides (GM2, GM3); Mycobacterium glycolipids; PGL-1; Ag85B; TBGL; Gonococcl lip-oligosaccharide epitope 2C7 from Neisseria gonorrhoeae; Lewis(y); Globo-H; Tn; TF; STn; PorA; TspA or Viral glycolipids/glycoproteins and surface proteins; and the like.

The antigen molecule may be in the form of a peptide antigen or it may be a nucleic acid encoding an antigenic peptide in a form suitable for expression in the treated mammal and presentation to the immune system. The antigen may also be a glycolipid or a glycopeptide. In any case, the antigen may be a complete antigen, or it may be a fragment of a complete antigen including at least one therapeutically relevant epitope. As used in this application, the term "therapeutically relevant epitope" refers to epitopes for which the mounting of an immune response against the epitopes will provide a therapeutic benefit. Thus, this term would exclude fragments which might be highly immunogenic, but which do not produce an immune response directed at the complete antigen or antigenic source (for example a bacteria). Combination antigens which include multiple epitopes from the same target antigen or epitopes from two or more different target antigens (polytope vaccines). In the latter case, the antigens can be of the same or different types (for example peptide + peptide, glycolipid + peptide, glycolipid + glycolipid.

The vaccine composition of the invention comprise a lipid-nucleic acid particle and an antigenic molecule. In a preferred embodiment of the invention, the antigenic molecule is associated with the lipid-nucleic acid particle.

It bears mention that the vaccines of the present invention may be administered by intramuscular or subcutaneous injection. This reduces some of the manufacturing constraints which are desirable when making composition for intravenous administration. In particular, larger-sized (150-300 nm) lipid particles can be used, which can eliminate or reduce the need for costly extrusion steps. Further, because the particles do not need to circulate, the selection of lipid components can be biased in favor of less expensive materials. For example, the amount of Chol can be reduced, DSPC can be replaced with something less rigid, such as DOPC or DMPC) and PEG-lipids can be replaced with less expensive PEG-acyl chains.

B. Manufacturing of Compositions

B.1 Manufacturing

Manufacturing and preparation of the compositions of the invention may be accomplished by any technique, but most preferred are the ethanol dialysis or detergent dialysis methods detailed in the following publications and patent applications, all incorporated herein by reference: US Pat. No. 5,705,385; US Pat. Applic. S.N. 08/660,025; 09/140,476; 08/484,282; 08/856,374; 09/078954; 09/078955; 60/143,978; and PCT Publication Nos. WO 96/40964 and WO 98/51278.

These methods provide for small and large scale manufacturing of lipid-nucleic acid particles, and generate particles with excellent pharmaceutical characteristics (described in C.2, infra). Certain specific embodiments of these techniques are set out in the examples below.

In addition to detergent dialysis and ethanol dialysis techniques, classical liposome manufacturing techniques may be employed to generate particles of the invention, albeit with greater difficulty. Traditional techniques of passive loading, active loading (by pH gradient), lipid film rehydration, extrusion/sizing, dehydration, etc. are amply set out elsewhere in the art, including the above noted patent documents.

These classical techniques are likely to be used when incorporating additional, conventional therapeutic agents into the compositions of the invention. The loading of tertiary or quaternary amine containing cytotoxic compounds such as doxorubicin, daunorubicin, vinca alkaloids, such as vincristine and vinblastine, can be achieved after formulation of the lipid-nucleic acid particle. Conveniently, the interior space of the particle will retain the low pH 4.2 of the original formulation procedure. Simple addition of the therapeutic agent in neutral buffer solution to a neutralized particle mixture is sufficient to load the particles, as is well known in the art.

Vaccine compositions of the invention may be prepared by adding the weak antigen to which the response is desired during the formulation process, or by post-formulation manipulations. Means of incorporating antigens include: 1. Passive encapsulation during formulation process (i.e. put in with ODN solution); 2. For glycolipids and other antigenic lipids, incorporate into ethanol mixture of lipids and formulate as per preferred protocols; 3. Post insertion (i.e antigen-lipid can be added into formed vesicles by incubating the vesicles with antigen-lipid micelles); and 4. Post-Coupling in which a lipid with a linker moiety is included into the formulated particle, and the linker is activated post formulation to couple the desired antigen. Standard coupling and cross-linking methodologies are known in the art. An alternative preparation incorporates the antigen into a lipid-particle which does not contain a nucleic acid, and these particles are mixed with lipid-nucleic acid particles prior to administration to the patient.

B.2 Characterization of compositions of the invention.

Regardless of the technique employed for their manufacture, the compositions of the invention have the following preferred characteristics.

The lipid-nucleic acid particles of the invention comprise a lipid membrane (generally a phospholipid bilayer) exterior which fully encapsulates an interior space. These particles, also sometimes herein called lipid membrane vesicles, are small particles with mean diameter 50-200 nm, preferably 60-130 nm. Most preferred for intravenous administrations are particles are of a relatively uniform size wherein 95% of particles are within 30 nm of the mean. The nucleic acid and other bioactive agents are contained in the interior space, or associated with an interior surface of the encapsulating membrane.

"Fully encapsulated" means that the nucleic acid in the particles is not significantly degraded after exposure to serum or a nuclease assay that would significantly degrade free DNA. In a fully encapsulated system, preferably less than 25% of particle nucleic acid is degraded in a treatment that would normally degrade 100% of free nucleic acid, more preferably less than 10% and most preferably less than 5% of the particle nucleic acid is degraded. Alternatively, full encapsulation may be determined by an Oligreen™ assay. Fully encapsulated also suggests that the particles are serum stable, that is, that they do not rapidly decompose into their component parts upon *in vivo* administration.

These characteristics distinguish the key particles of the invention from lipid-nucleic acid aggregates (also known as cationic complexes or lipoplexes) such as DOTMA/DOPE (LIPOFECTIN™) formulations. These aggregates are generally much larger (>250 nm) diameter, they do not competently withstand nuclease digestion, and they generally decompose upon *in vivo* administration. Formulations of cationic lipid-nucleic acid aggregates with weak antigens, as described above, may provide suitable vaccines for local and regional applications, such as intra-muscular, intra-peritoneal and intrathecal administrations.

The particles of the invention can be formulated at a wide range of drug:lipid ratios. As used herein, "drug to lipid ratio" means the amount of therapeutic nucleic acid (i.e. the amount of nucleic acid which is encapsulated and which will not be rapidly degraded upon exposure to the blood) in a defined volume of preparation divided by the amount of lipid in the same volume. This may be determined on a mole per mole basis or on a weight per weight basis, or on a weight per mole basis. Drug to lipid ratio determines the lipid dose that is associated with a given dose of nucleic acid; note that the highest possible drug to lipid ratio is not always the most potent formulation. Particles of the invention are useful in the range of 0.001 to 0.45 drug:lipid ratio (w/w).

Vaccine compositions are similar to other particles of the invention, except by having the weak antigen associated (either covalently or non-covalently) with the particle.

C. Uses of Lipid-Nucleic Acid Compositions

In its broadest sense, the invention described herein relates to compositions of lipid formulated nucleic acids and their methods of use for inducing an immune response in a mammal. There are several remarkable and surprising advantages of the invention over prior art uses of immune stimulating nucleic acids, including:

1. Compared to free formulations of nucleic acids, the lipid formulations employed deliver the nucleic acids to different cells and immune system components, and present them in a different fashion to these cells and components, thus rendering significantly different and improved immune responses, some of which are illustrated in the examples below;

2. Compared to free formulations of nucleic acids, lipid formulations require significantly lower amounts of oligonucleotide to render an immune response, thus reducing cost and potential toxicities;

3. Lipid formulations can deliver phosphodiester oligonucleotides for use in immune stimulation, a chemistry which can not be delivered in the free form.

4. Lipid formulations can convert normally non-ISS nucleic acids into ISS nucleic acids, thus creating a new class of ISS.

5. A more potent vaccine can be generated in liposomal formulations of nucleic acids, because weak immunogens to which an immune response is desired can be directly and inherently associated with the formulation, thus leading to different and improved immune responses to the immunogens, as opposed to simple mixing of adjuvants and immunogens (as found in PCT publication WO 98/40100, Inventor: Davis et al.);

6. By using lipid formulations which are known to be useful in obtaining direct "antisense" effects with nucleic acids (see, : US Pat. No. 5,705,385; US Pat. Applic. S.N. 08/660,025; 09/140,476; 08/484,282; 08/856,374; 09/078954; 09/078955; 60/143,978; and PCT Publication Nos. WO 96/40964 and WO 98/51278, which are incorporated herein by reference), the formulations of the invention can result in synergistic immune response and antisense effects which combine to treat disorders.

7. Co-administration of lipid formulations containing nucleic acids and a cytotoxic agent such as doxorubicin can result in synergistic immune response and cytotoxic effects which combine to treat disorders.

8. Lipid formulations of nucleic acids have demonstrated therapeutic efficacy in *in vivo* models which do not respond to free form ISS nucleic acids.

Each of these advantages is illustrated in one or more examples set out below.

"Immune stimulation" or "inducing an immune response" is broadly characterized as a direct or indirect response of an immune system cell or component to an intervention. These responses can be measured in many ways including activation, proliferation or differentiation of immune system cells (B cells, T cells, dendritic cells, APCs, macrophages, NK cells, NKT cells etc.), up-regulated or down-regulated expression of markers, cytokine, interferon, IgM and IgG release in the serum, splenomegaly (including increased spleen cellularity), hyperplasia and mixed cellular infiltrates in various organs. Many more responses and many other immune system cells and components are known in the art. Further, the stimulation or response may be of innate immune system cells, or of the acquired immune system cells (for example, as by a vaccine containing a normally weak antigen.) Immune stimulation is distinguishable on a mechanistic basis from other potential effects of nucleic acids, such as direct antisense effects (through hybridization with mRNA) or gene expression (as by plasmids), however, in the result, the desired consequence of therapeutic efficacy is not necessarily distinguishable.

### D. Indications, Administration and Dosages

Among other things, the compositions and methods of the invention are indicated for use in any patient or organism having a need for immune system stimulation. This can include most medical fields, such as oncology, inflammation, arthritis & rheumatology, immuno-deficiency disorders, etc. One skilled in the art can select appropriate indications to test for efficacy based on the disclosure herein. In a preferred embodiment, the compositions and methods of the invention are used to treat a neoplasia (any neoplastic cell growth which is pathological or potentially pathological) such as the neoplasia described in the Examples below.

Administration of the compositions of the invention to a subject/patient may be by any method including *in vivo* or *ex vivo* methods. *In* vivo methods can include local, regional or systemic applications. In a preferred embodiment, the compositions are administered intravenously such that particles are accessible to B cells, macrophages or a splenocytes in a patient, and/or the particle can stimulate lymphocyte proliferation, resulting in secretion of IL-6, IL-12, IFNγ and/or IgM in said patient.

One skilled in the art knows to identify possible toxicities of formulations such as complement activation, coagulation, renal toxicities, liver enzyme assays, etc. Such toxicities may differ between organisms. In the examples below, toxicities are reported if identified; no toxicities were observed in rodents for up to 600 mg/kg lipid doses (except where identified in repeat dosing situations).

Pharmaceutical preparations of compositions usually employ additional carriers to improve or assist the delivery modality. Typically, compositions of the invention will be administered in a physiologically-acceptable carrier such as normal saline or phosphate buffer selected in accordance with standard pharmaceutical practice. Other suitable carriers include water, 0.9% saline, 0.3% glycine, and the like, including glycoproteins for enhanced stability, such as albumin, lipoprotein, globulin, etc.

Dosages of lipid-nucleic acid particles depend on the desired lipid dosage, the desired nucleic acid dosage, and the drug:lipid ratio of the composition. In mammals, a typical lipid dose is between 0.5 mg/kg and 300 mg/kg. A large amount of lipid is immediately cleared by the RES cells (such as Kupffer cells of the liver) upon administration, thus a minimum lipid dosage is generally required to saturate the RES and allow particles to circulate. Dosages of nucleic acid are preferably between 0.01 mg/kg and 60 mg/kg. Typically, a mammal will receive a formulation of drug:lipid ratio 0.01 to 0.25, and will therefore receive 100 mg/kg lipid and 1-25 mg/kg nucleic acid. Primate doses typically will be 5 - 50 mg/kg lipid and 0.005 -15 mg/kg ODN. One skilled in the art can select proper dosages based on the information provided herein.

E. Examples:

Materials & Methods

***Oligodeoxynucleotide ("ODN") and Plasmid DNA.*** The designations and 5'-3' sequences of the ODN (with known descriptions contained in parentheses) and plasmid used in these studies were as follows:

hICAM or INX-2302 (3' untranslated region of human ICAM-1 mRNA) (PO & PS);
GCCCAAGCTGGCATCCGTCA                                                                SEQ ID No. 1
mICAM or INX-3082 (3' untranslated region of murine ICAM-1 mRNA) (PO & PS);
TGCATCCCCCAGGCCACCAT                                                                SEQ ID No. 2
EGFR (human epidermal growth factor mRNA, receptor translation termination codon region);
CCGTGGTCATGCTCC                                                                SEQ ID No. 3
c-myc or INX-6295 (initiation codon region of human/mouse c-myc proto-oncogene mRNA) (PS and methylated PS);
TAACGTTGAGGGGCAT                                                                SEQ ID No. 4
c-myc or INX-3280 or LR-3280 (initiation codon region of human/mouse c-myc proto-oncogene mRNA) (PS);
AACGTTGAGGGGCAT                                                                SEQ ID No.5
c-myc or INX-6298 (initiation codon region of human/mouse c-myc proto-oncogene mRNA) (PO & PS);
AACGTTGAGGGGCAT                                                                SEQ ID No. 5
c-mycC or INX-6300 (non-ISS control similar composition to INX-6295)
TAAGCATACGGGGTGT                                                                SEQ ID No. 6
LR-4420 (ISS control similar composition to INX-6295)
AACGAGTTGGGGCAT                                                                SEQ ID No. 7
LR-3001 or INX-3001 (hybridizes to c-myb mRNA);
TATGCTGTGCCGGGGTCTTCGGGC                                                                SEQ ID No. 8
IGF-1R or INX-4437 (hybridizes to IGF-1R mRNA);
GGACCCTCCTCCGGAGCC                                                                SEQ ID No. 9
INX-6299 (control PO for INX-6298)
AAGCATACGGGGTGT                                                                SEQ ID No.10
INX- 8997 (Control containing 3 CpG motifs) (PO & PS)
TCGCATCGACCCGCCCACTA                                                                SEQ ID No.11

Plasmid DNA employed was the luciferase expression plasmid, pCMVluc18, (also called pCMVLuc). Plasmid was produced in *E. Coli,* isolated and purified as described previously (Wheeler, J. J., Palmer, L., Ossanlou, M., MacLachlan, I., Graham, R. W., Zhang, Y. P., Hope, M. J., Scherrer, P., & Cullis, P. R. (1999) Gene Ther. 6, 271-281.). (See also Mortimer I, Tam P, MacLachlan I, Graham RW, Saravolac EG, Joshi PB. Cationic lipid mediated transfection of cells in culture requires mitotic activity. Gene Ther. 1999;6: 403-411.).

Phosphodiester (PO) and phosphorothioate (PS) ODN were purchased from Hybridon Specialty Products (Milford, MA) or were synthesized at Inex Pharmaceuticals (Burnaby, BC, Canada). Methylated ODN were manufactured by standard techniques at Inex Pharmacueticals (USA), Inc. (Hayward, CA). The backbone composition was confirmed by $^{31}$P-NMR. All ODN were specifically analyzed for endotoxin and contained less than 0.05 EU/mg.

Example 1

This series of examples illustrates, among other things, that sterically-stabilized liposomes containing polyethylene glycol-lipid conjugates are immunogenic when the liposomes contain nucleic acid, and identifies uses of such compositions.

***Chemicals and Lipids.*** DSPC (1,2-distearoyl-sn-glycero-3-phosphocholine) and polyethylene glycol conjugated-distearoylphosphatidylethanolamine (PEG$_{2000}$-DSPE) were purchased from Avanti Polar Lipids (Pelham, AL) or Northern Lipids (Vancouver, BC, Canada). Cholesterol (CH) was purchased from Sigma (St. Louis, MO). 1,2-dioleoyl-3-N,N-dimethylammoniumpropane (DODAP) was synthesized by Dr. Steven Ansell (Inex Pharmaceuticals Corp.) or, alternatively, was purchased from Avanti Polar Lipids (DODAP only). 1-O-(2'-(ω-methoxypolyethyleneglycol)succinoyl)-2-N-myristoylsphingosine (PEG-CerC$_{14}$) and 1-O-(2'-(ω-methoxypolyethyleneglycol)succinoyl)-2-N-arachidoylsphingosine (PEG-CerC$_{20}$) were synthesized by Dr. Zhao Wang (Inex Pharmaceuticals Corp.). [$^3$H]-cholesterylhexadecylether (CHE) was obtained from Dupont NEN (Boston, MA). All lipids were >99% pure. All reagents were used without further purification.

*Encapsulation of ODN & Plasmid.* Stabilized antisense-lipid particles (SALP) composed of DSPC:CH:DO-DAP:PEG-CerC$_{14}$ (sometimes called AS4200) or DSPC:CH:DODAP:PEG-CerC$_{20}$ (sometimes called AS4204) and encapsulated PS ODN were prepared as described in the parent patent applications of the instant patent application, namely US Patent Applications Serial Nos. 08/856,3 74, 09/078954, 09/078955 and PCT Publication WO 98/51278, all assigned to the assignee of the instant patent application, and incorporated herein by reference. Typically 1000 mg of total lipid was dissolved in 100 ml of ethanol. A solution containing the ODN was prepared in a separate flask by dissolving 200mg (based on A$_{260}$) in 60 mls of 300 mM citric acid, pH 4.0. The lipid solution was added to the ODN solution dropwise through a 26G needle while stirring constantly. The mixture was passed 10 times through 2 stacked, 80 nm polycarbonate filters (Poretics) using a thermobarrel extruder (Lipex Biomembranes, Vancouver, BC, Canada) maintained at 65˚C. The citrate buffer was exchanged with 20 volumes of 20mM PBS/145mM NaCl using a tangential flow apparatus with a 100 000 M.W. cut-off. This step removes excess ethanol and unencapsulated ODN and generates an isotonic solution compatible with *in vivo* administration. The SALP preparation was concentrated using tangential flow, adjusted to 1.5 mg/ml ODN, filter-sterilized through a 0.22 $\mu$M membrane and stored at 4˚C. SALP mean diameter and size distribution was determined using a NICOMP Model 370 Sub-micron particle sizer and was typically 110 $\pm$ 30 nm. Where formulations containing a lower oligonucleotide: lipid ratio by weight were required, the ODN concentration of the initial solution was reduced by the appropriate ratio to generate the particles, as further described in the parent cases of the instant application. Alternatively, for administration, some samples were switched to HEPES-buffered saline (HBS), pH 7.50, and dialyzed for a minimum of 12 hours to replace the external citrate buffer with HBS. This renders the majority of DODAP in the outer bilayer neutral, and will release any surface bound antisense. Additional non-encapsulated antisense may optionally then removed from the AS4200/4 by DEAE-sepharose chromatography. For PO-ODN and plasmid formulations, initial buffer employed is 20 mM citrate, pH 4.0. Plasmid formulations were not extruded, resulting in ~200 nm particles. ODN encapsulated in AS4200 or AS4204 formulations are sometimes herein referred to by the ODN name but changing INX to INXC (i.e. INXC-6295)

*Control formulations* were prepared by standard liposome methods known in the art: DSPC:CH and DSPC:CH:PEG$_{2000}$-DSPE vesicles were prepared from dry lipid films by aqueous hydration in HBS (20 mM Hepes, 145 mM NaCl, pH 7.4), according to the method of Hope *et al.* (41). Similarly, ODN encapsulation was achieved by hydration of 100 mg of lipid with 100 mg of ODN in 1.0 ml HBS, followed by 5 cycles of freezing-thawing and extrusion through 2 stacked 100 nm filters. [$^3$H]-CHE, a non-exchangeable, non-metabolizable lipid marker was incorporated into all vesicle compositions to monitor lipid levels in the blood (42). The resulting particles were approximately 110-140 nm in diameter as judged by quasi-elastic light scattering using a NICOMP Submicron particle sizer (Model 370). Encapsulation efficiencies for this process were typically less than 10%. Non-encapsulated ODN was removed from the preparation by anion exchange chromatography using DEAE-sepharose CL-6B. Free oligonucleotide is dissolved in HBS and adjusted to the required dose by A$_{260}$ (assuming 35 $\mu$g/ml gives and A$_{260}$ of 1.0). Where doxorubicin is co-encapsulated with oligonucleotide, the oligonucleotide containing particle is first prepared according to these methods, then the doxorubicin is loaded into the particle to the desired concentration, using standard pH loading techniques. Doxorubicin blockade experiments were performed using DSPC/Chol encapsulated doxorubicin (~10 mg/ lipid/kg and 0.05 to 0.2 mg Dox/kg) prepared by pH loading, and administered to mice 24 hours prior to injecting AS4204.

*Mice.* Female, 7-8 week old ICR, C57BL/6 and Balb/c mice were obtained from Harlan Sprague Dawley (Indianapolis, IN). Balb/c *nu/nu* and Balb/c SCID-Rag2 mice were obtained from The Jackson Laboratory (Bar Harbor, ME) and were maintained under pathogen-free conditions. All animals were quarantined for at least one week prior to use. All procedures involving animals were performed in accordance with the guidelines established by the Canadian Council on Animal Care.

*Dosages:* Mice were dosed every other day for the duration of the study (7 or 10 doses total as indicated) unless otherwise indicated. Administrations of test samples and controls were *via* intravenous tail vein injections (injection volume: 200 $\mu$l). Unless otherwise indicated, lipid dose for these formulations is adjusted to 100 mg/kg/dose. In experiments where different drug:lipid ratios are employed, lipid dose for all formulations was adjusted to 80 mg/kg/dose. Samples are filtered (0.22 $\mu$m) prior to injection. External buffer is HBS (20mM Hepes, 145 mM NaCl, pH 7.45).

*Liposome Elimination from the Circulation.* For estimations of liposome elimination from the blood (also herein called "Liposome Recovery in Blood"), mice received a single intravenous dose, via the lateral tail vein, of empty liposomes (50 mg/kg lipid) or liposome-encapsulated ODN (50 mg/kg lipid and 20 mg/kg ODN, unless otherwise noted) containing ~ 1 $\mu$Ci/mouse of [$^3$H]-CHE. Dosing was weekly unless otherwise noted. Blood (25 $\mu$l) was collected at 1 h post-injection by tail nicking using a sterile scalpel and placed in 200 $\mu$l of 5% EDTA in a glass scintillation vial. The blood was then digested (Solvable™, Packard), decolorized and analyzed for radioactivity using standard liquid scintillation techniques according to the manufacturer's instructions. The tail nicking procedure yielded very similar results to groups of mice that had blood sampled by cardiac puncture, but was more useful because all data was collected from the same group of animals.

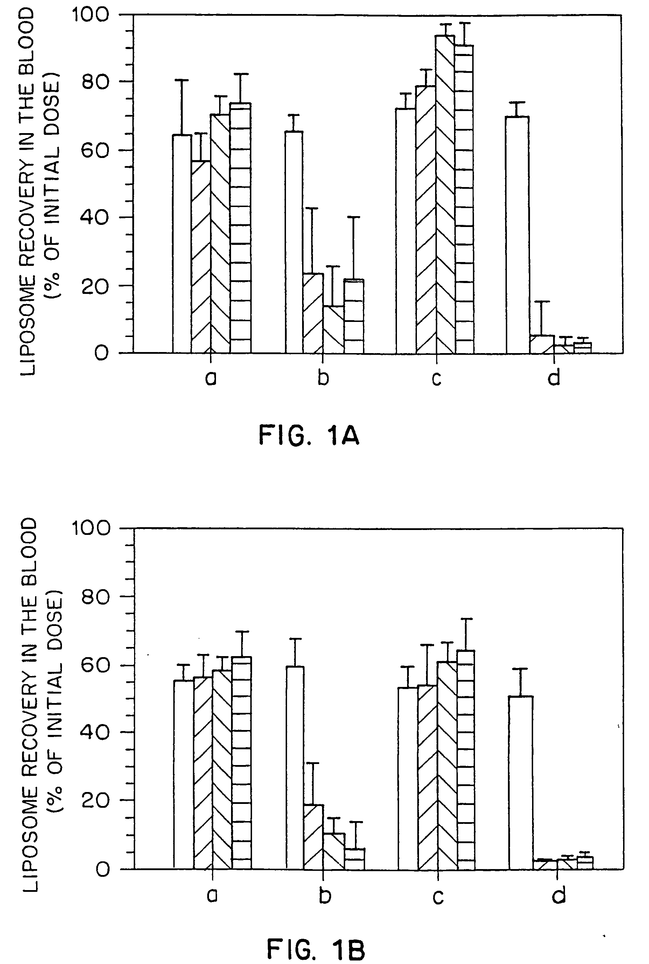

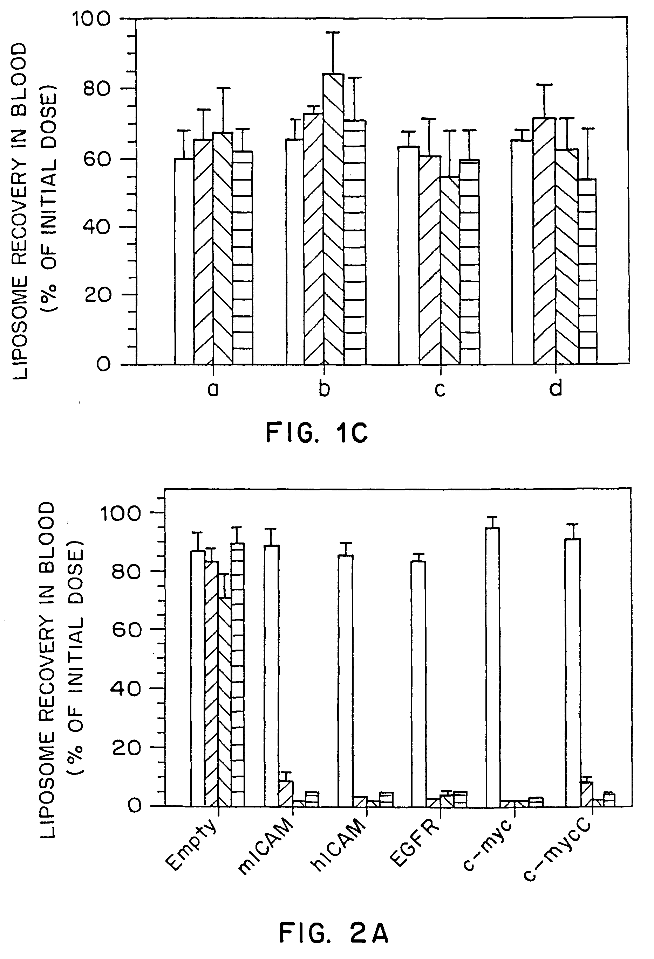

**Figures 1 A-C** demonstrate circulation levels of PEG-liposomes on repeat administration in immune competent Balb/c mice (Fig. 1A), and immune compromised Balb/c nude (Fig. 1B) and Balb/c SCID-Rag2 mice (Fig. 1C). Mice were injected intravenously (i.v) with empty DSPC:CH:PEG$_{2000}$-DSPE liposomes (a), DSPC:CH: PEG$_{2000}$-DSPE liposomes containing hICAM PS ODN (b), empty SALP (PEG-CerC$_{20}$, c), or SALP (PEG-CerC$_{20}$, d) containing hICAM ODN. Lipid doses were 50 mg/kg. The ODN/lipid ratio for the DSPC:CH:PEG$_{2000}$ and SALP (PEG-CerC$_{20}$) were 0.05 and 0.20, respectively. Injections were administered weekly and the circulation levels at 1 h post-injection were monitored by the lipid label [3H]-CHE. The bars represent the first (open bars), second (back slash), third (forward slash) and fourth (cross-hatched) injection. All bars represent the mean and standard deviation of 8 mice. As reflected in the "a" and "c" columns, no differences in elimination were observed for empty PEG-lipid containing vesicles over several administrations, regardless of immune status of animals. However, surprising and rapid elimination (<20% of injected dose remained in the blood at 1h) of ODN-containing vesicles was observed following the second and subsequent injections. This effect was accompanied by pronounced morbidity and, in some instances, resulted in death of the animal within 30 minutes post-injection. This rapid elimination was also observed in T-cell deficient Balb/c nude mice, but not in B-cell and T-cell deficient Balb/c SCID-Rag2 mice, establishing that the response is dependent on the presence of B-cells and immunoglobulin.

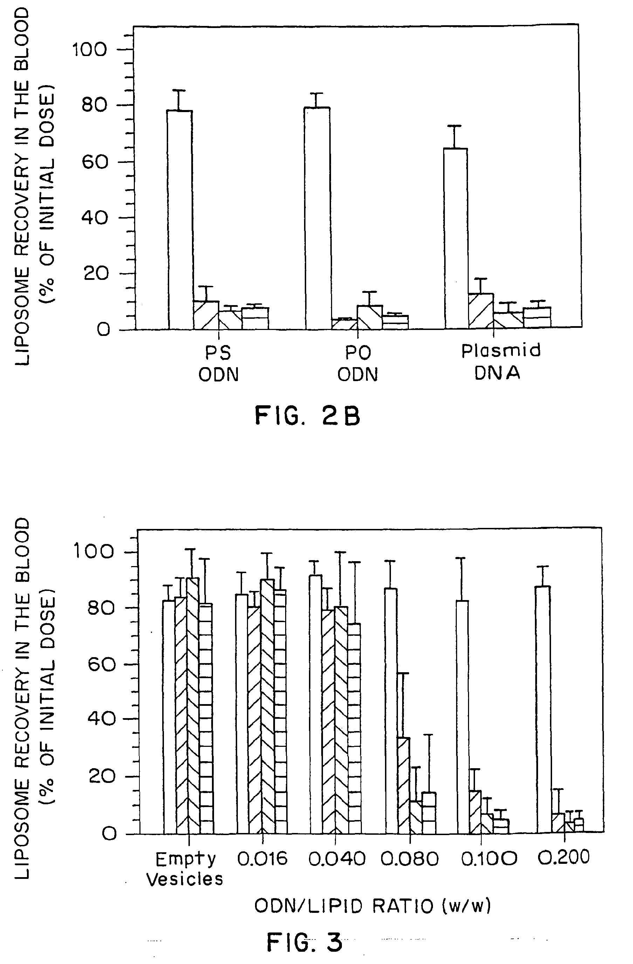

**Figure 2 A and B** show the influence of nucleic acid sequence (A) and structure (B) on elimination of SALP (PEG-CerC$_{20}$). Mice were injected i.v. with SALP (PEG-CerC$_{20}$) containing PS ODN of various nucleotide sequences (Fig 2A). Phosphodiester (PO) hICAM ODN and bacterial plasmid DNA were also evaluated (Fig. 2B). The lipid dose was adjusted to 50 mg/kg and the ODN/lipid ratio for each formulation was ~ 0.20. Injections were administered weekly and the circulation levels at 1 h post-injection were monitored by the lipid label [3H]-CHE. The bars and numbers of animals are indicated as in the legend to Figures 1 A-C. Following i.v. administration, all PS ODN encapsulated in SALP (PEG-Cer C20) induced morbidity and were rapidly removed from the circulation upon repeat administrations. This was observed regardless of ISS (hICAM, c-myc, c-mycC) or non-ISS (i.e. mICAM, EGFR) status of ODN. **Figure 2B** shows that rapid elimination of the particle from the blood ensues regardless of whether the encapsulated nucleic acid was PS, P0 or plasmid (weekly injections monitored at 1 h post injection).

**Figure 3** shows the relationship between DNA to lipid ratio and liposome recovery. Mice were injected i.v. with SALP (PEG-CerC$_{20}$) containing hICAM PS ODN at various ODN/lipid ratios. The lipid dose was adjusted to 50 mg/kg/ dose. Injections were administered weekly, and the circulation levels at 1 h post-injection were monitored by the lipid label [3H]-CHE. The bars and numbers of animals are indicated are in the legend to Figures 1 A-C. As shown, the results demonstrate that particles containing greater than 0.040 drug:lipid ratio (w/w) induce the rapid clearance response, while particles of 0.040 or less are not subject to clearance upon repeat injection. This result suggests that the immune system recognizes a threshold amount (or concentration) of nucleic acid before mounting the clearance response. Also, particles below 0.040 w/w are useful for obtaining direct antisense effects and will evade the rapid clearance response upon repeat administration in the AS4204 (long circulating) formulation.

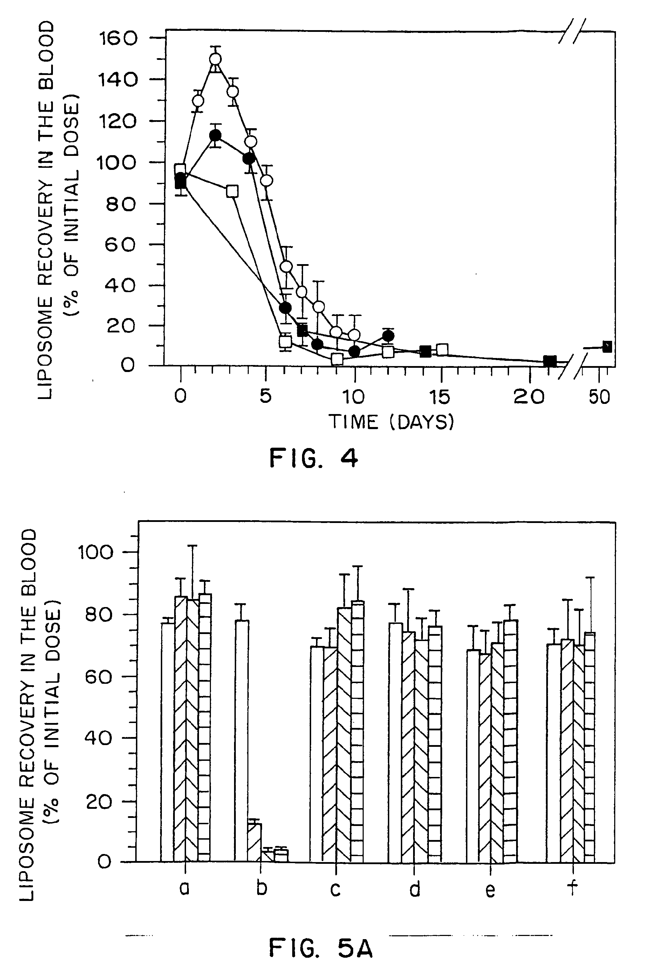

**Figure 4** shows the influence of administration schedule on the onset of the rapid elimination response. Mice were injected i.v. with SALP (PEG-CerC$_{20}$) containing hICAM PS ODN at various dosing schedules: daily (O), every 2 days (•), every 3 days (□) and weekly (■). The lipid dose was adjusted to 50 mg/kg/dose and the circulation levels at 1h post-injection were monitored by the lipid label [3H]-CHE. The symbols represent the mean and standard deviation of 8 mice. D:L ratios of particles are above the threshold clearance inducing levels. As shown in Figure 4, it takes at least 5 days for rapid clearance response capacity to develop in a mouse, regardless of how often the SALP is administered (daily, every 2, 3, or 7 days). For daily injections, the plasma levels of circulating carrier increased over the first 3 injections. This was not surprising given that 30-40% of a given dose of PEG-coated liposomes remains in the circulation at 24 h post-injection. However, this increase was followed by a dramatic decline in the circulation levels of subsequent doses. In all dosing schedules, rapid elimination of subsequent doses was observed 4-6 days after the initial dose. These results establishes that immune system components mounting the clearance response are saturated after an initial dose, and that processing and generation of clearance response takes approximately 5-6 days, regardless of the number of intervening doses. This suggests a humoral (Ab) response is being generated.

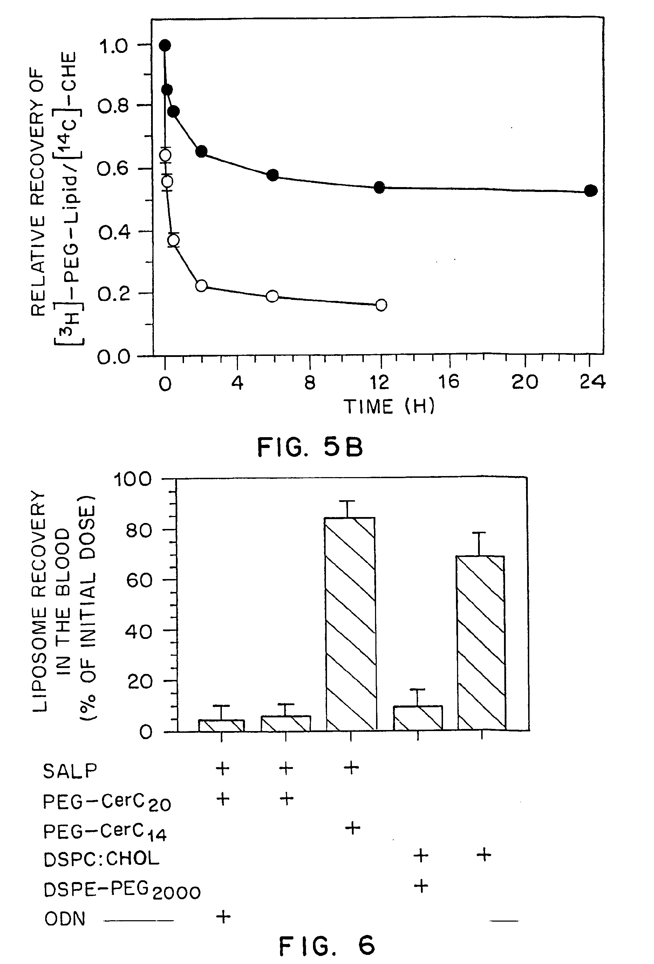

**Figures 5 A and B** illustrate the role of PEG-lipid in the rapid elimination of liposomes containing ODN. In a first experiment, reported in Fig. 5A, mice were injected i.v. with empty SALP (PEG-CerC$_{20}$, a), SALP (PEG-CerC$_{20}$, b), empty SALP (PEG-CerC$_{14}$, c), SALP (PEG-CerC$_{14}$, d), empty DSPC:CH liposomes (e) or DSPC:CH containing hICAM PS ODN (f). The lipid dose was adjusted to 50 mg/kg/dose and the circulation levels at 1h post-injection were monitored by the lipid label [3H]-CHE. The bars and numbers of animals are indicated in the legend to Figure 1. In a second experiment reported in Fig. 5B, the time course for exchange of PEG-CerC$_{20}$ (•) and PEG-CerC$_{14}$ (○) was evaluated by monitoring the ratio of [3H]-PEG-ceramide to [14C]-CHE in the plasma of mice over 24 h. The symbols represent the mean and standard deviation of 6 mice.

PEG-CerC14 has a shorter acyl chain lipid-anchor than PEG-CerC20, and therefore more readily exchanges out of the bilayer ($t_{1/2}$ *in vivo* = ~ 3 min *vs.* >*24 h).* When PEG-CerC 14 is employed in particles of the invention, no rapid clearance response is detected upon repeat administration (columns c & d) compared to CerC20 containing SALPs (column b). Since neither empty nor ODN carrying DSPC:CH vesicles nor PEG-CerC14 formulations exhibit any differences, we conclude that the presence and retention of PEG-lipid in the external monolayer of the vesicles was critical for development of the rapid clearance response.

**Figure 6** the results of cross-over studies conducted after 3 previous weekly injections of ODN containing PEG-CerC20 SALPs had initiated the clearance response. Mice were injected i.v. with SALP (PEG-CerC$_{20}$) for a total of 4 weekly injections, resulting in an elimination profile similar to that observed in Figures 1 A-C. On the final injection, mice received either SALP (PEG-CerC$_{20}$), empty SALP (PEG-CerC$_{20}$), empty DSPC:CH:PEG$_{2000}$-DSPE vesicles, empty SALP (PEG-CerC$_{14}$) or empty DSPC:CH liposomes. In each instance, the lipid dose was adjusted to 50 mg/kg/dose and the circulation levels at 1 h post-injection were monitored by the lipid label [$^3$H]-CHE. Each bar represents the mean and standard deviation of 8 mice. The fourth administration demonstrated that the clearance response was directed exclusively to those particles where PEG-lipid is retained in the outer lipid monolayer, regardless of whether they carry ODN. Thus PEG-CerC20 and PEG-DSPE formulations are cleared, regardless of ODN status, whereas non-PEG-lipid formulations (such as DSPC:CH) or exchangeable PEG-lipid formulations (such as PEG-CerC14) are not cleared. This establishes that the clearance response does not depend on the (interior) ODN status of the particle, once it has learned to recognize the formerly weak immunogen on the external surface of the particle. This result suggests a wide variety of synthetic liposomal vaccines could be generated according to this invention, which include weak immunogens on the exterior surface of the particle. The vaccine would first be administered in ODN containing format, and subsequent challenge to the patient by a pathogen would be recognized regardless of ODN status of the pathogen.

Example 2

This series of examples illustrates further responses to immune stimulating lipid-nucleic acid particles. These methods employ the materials and methods of Example 1, with the following changes.

The following mouse strains were used in these studies: ICR, Balb/c, Balb/c Nude, Balb/c SCID-Rag2, C57BL/6. All are commercially available from Harlan Sprague Dawley (Indianapolis, IN) or Taconic Farms (Germantown, NY).

Tumor models in these mice were established as follows.

*B16/BL6 Murine Melanoma.* Cells [NCI catalog B 16BL-6] were maintained in culture in MEM media supplemented with 10% FBS. On day 0 of the study, $3 \times 10^5$ cells were injected sub-cutaneously (s.c.) into the dorsal flank (injection volume: 50 $\mu$l) of C57BL/6 female mice (20-23g). Typically, 15% extra mice were injected so non-spheroidal tumours or mice in which no tumours were observed could be excluded from the study. Tumours were allowed to grow for a period of 5-7 days prior to initiating treatments with test samples/controls and randomly grouped. Treatment began when tumours were 50-100 mm$^3$.

*DoHH2 human follicular lymphoma.* DoHH2 cells (a non-Hodgkin's B-cell lymphoma cell line described in Kluin-Nelemans HC, et al. (1991) Leukemia 5(3) 221-224) are maintained in culture in RPMI 1640 media supplemented with 10% FBS. On day 0 of the study, $5 \times 10^6$ cells are injected intravenously (i.v.; injection volume, 200 $\mu$l HBSS) in SCID/Rag-2 female mice (20-23 g). Tumours are allowed to grow for a period of 3 days prior to initiating treatments with test samples/controls. On day 3, mice are randomly grouped prior to administrations.

*Lewis Lung.* Murine Lewis lung carcinoma cells (ATCC # CRL-1642) were grown in MEM media supplemented with 10% FBS. On day 0 of the study, $3 \times 10^5$ cells were injected sub-cutaneously (s.c.) into the dorsum (injection volume: 100 $\mu$l). Tumours were allowed to grow for a period of 3 days prior to initiating treatments with test samples/controls. Primary tumour volume was measured using calipers.

*NG Melanoma.* A human primary melanoma [NG, Clark's level V], obtained from the biopsy of a patient at the Surgery Department of Regina Elena Cancer Institute (Rome, Italy), was employed as set out in Leonetti, C. et al. (1996) J. Nat. Canc. Inst. 88(7) 419-429). CD-1 male nude *(nu/nu)* mice, 6-8 weeks old, were injected in the hind leg muscles with a cell suspension of $2.5 \times 10^6$ NG cells. A tumour mass of~70 mg was evident in all mice on day 4 after implant. All experiments were carried out between the fifth and eighth passages of the NG tumour in nude mice.

*Potency/Efficacy Endpoints.* Results described herein as "increase in tumour size (or volume)" were measured as follows: Primary tumour volume was measured using calipers. Length (mm), width (mm) and height (mm) measurements were made every other day (on non-injection days) for the duration of the study. Tumour volumes were calculated from the formula:

$$\text{Tumour Volume (mm}^3) = (\pi/6)(L \times W \times H)$$

Mice were terminated (by $CO_2$ inhalation or cervical dislocation preceded by general anesthesia) when tumour volumes reached 10% of body weight or on the first signs of ulceration.

"Tumour Weight Inhibition" ("TWI %") is calculated as the mean tumour weight of treated groups divided by mean tumour weight of the control groups, minus 1 times 100). "Tumour Growth Delay" ("T-C") is calculated as median time in days for the treated (T) groups to reach an arbitrarily determined tumour weight (i.e. 250mg) minus median time in days for the control (C) group to reach the same size.

"Survival" or "% Survival" is calculated on the basis of the number of animals in the initial test group. In accordance with the guidelines of the Canadian Council on Animal Care, death was not used as an endpoint. Instead, animals were observed daily and euthanized at the first signs of morbidity or moribundity, which for these models was typically manifested as hind limb paralysis. In instances of euthanasia, death of the animal was recorded as the following day. *Other Endpoints:*

*IgM and IgG production/clearance:* IgM and IgG antibodies generated as an immune response to the compositions were measured by appropriate ELISA assays from blood samples collected from mice.

*Tumour Accumulation:* To monitor tumor accumulation of ODN, animals were injected intravenously (200 $\mu$l) with [$^3$H]-labeled ODN, either free or encapsulated in stabilized antisense-lipid particles (SALP). [$^{14}$C]-cholesterylhexa-decylether was incorporated into SALP as a non-exchangeable, non-metabolizeable lipid marker to monitor the fate of the delivery system. At various times, mice were euthanized and the tumors were surgically removed, weighed and placed in Fast Prep tubes. PBS (500 $\mu$l) was added to each tube and the sample were homogenized for 3 x 8 second using a Bio 101 Fast Prep FP120 apparatus (Savant). Aliquots (100-200 $\mu$l) of the tumor homogenate were then placed in 500 $\mu$l of tissue solubilizer (Solvable™, Packard) and digested and decolorized as per the manufacturer's instructions. The resulting samples added to 5.0 ml of Pico-Fluor40™ scintillation cocktail and were analyzed for total radioactivity using standard liquid scintillation methods. Results were expressed as $\mu$g ODN equivalents/g tissue.

*In vitro Splenocyte Proliferation Assay.* The mitogenicity of the ODNs used in these studies was evaluated by measuring stimulation of splenocyte proliferation in vitro. Splenocyte suspensions were prepared by gently teasing apart spleens in cRPMI using the frosted ends of two glass slides. Aliquots of 100 :1 of a freshly prepared splenocyte suspension ($5 \times 10^6$ cells/ml in completeRPMI) were added to triplicate wells of 96 well plates, containing an equal volume of complete RPMI with a 2X concentration of ODN (i.e.12.5, 25.0, 50.0, or 100.0 mg/ml ODN in complete RPMI). Twenty-four hours later, 1:Ci of [$^3$H]-thymidine (NEN Life Science Products; Boston, MA, USA) was added to each well and the cultures were incubated a further 48 h. At the end of the incubation period, cells were harvested onto glass filters and the quantity of incorporated radioactivity was measured using a beta scintillation counter. Appropriate controls (mitogens: ConA and LPS, or medium alone) were included on each plate. [$^3$H]-thymidine incorporation is expressed as the mean DPMs$\pm$SEM.

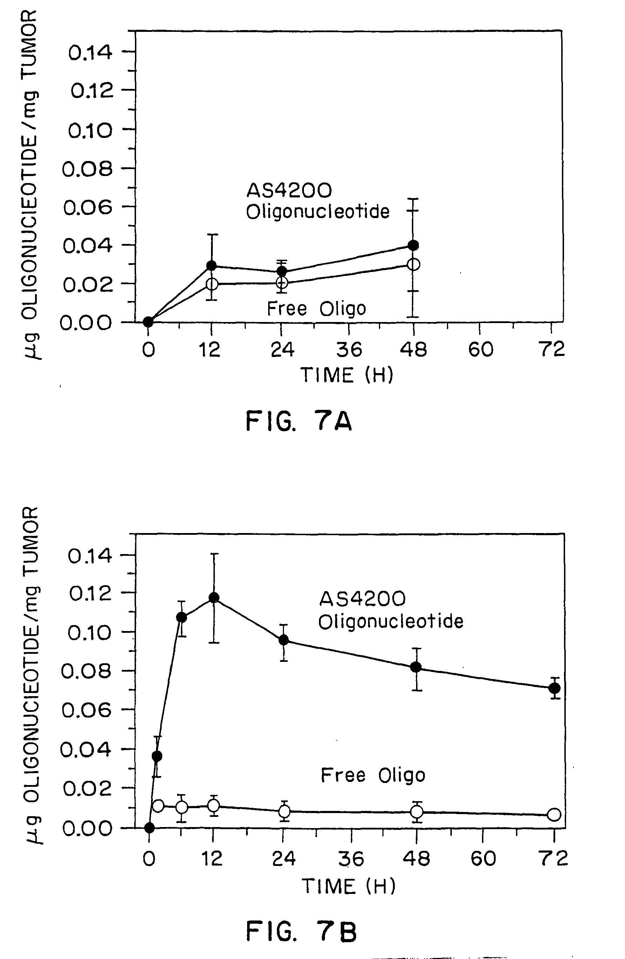

**Figures 7 A and B** demonstrates that the AS4200 formulation accumulates in certain solid tumours. AS4200 formulations were administered intravenously at time 0. Mice were sacrificed and tumours removed at indicated time points. ODN accumulation was measured. In Lewis lung tumours (Fig. 7B) it accumulates to a much higher degree than free ODN (5-10% of dose vs. 1% of dose); whereas in B16 tumours (Fig. 7A) it accumulates approximately the same amount as free ODN (1-3% of dose). ODN accumulation is influenced by the micro-environment of these tumours, and probably by the stage of tumour development.

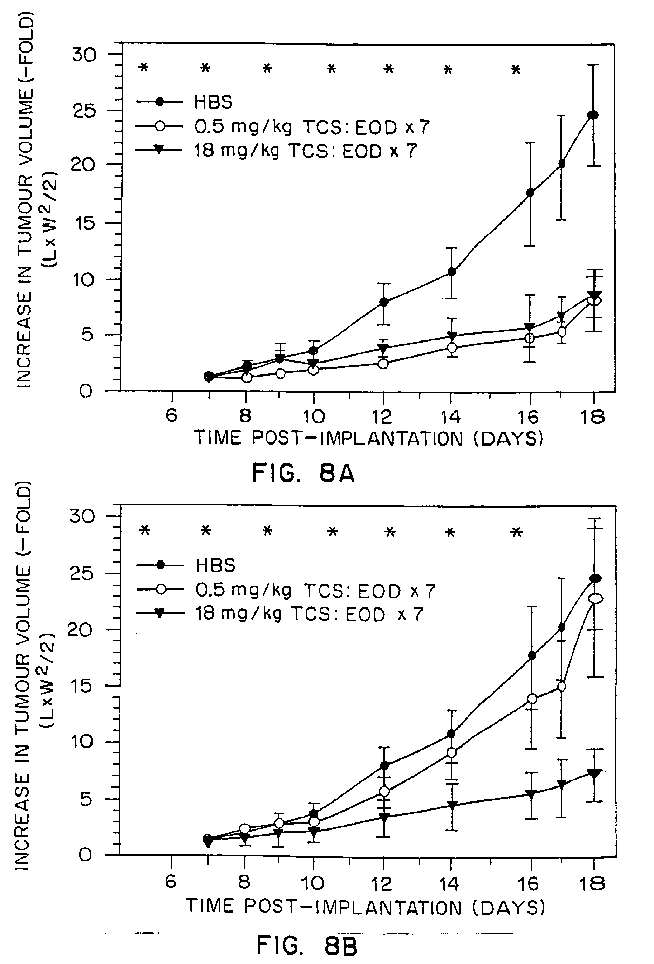

**Figures 8A and B** demonstrates that AS4200 greatly enhances the potency of 0DN. Treatments (lipid dose = 100 mg/kg; D/L ratio of 0.18 or 0.005) were administered every other day for 7 days starting on Day 5 post tumour implantation (identified by asterisks). "c-myc" = INX-6295. Tumour = B16 Murine melanoma. In the AS4200 formulation (Fig. 8A), an ODN dosage of 0.5 mg/kg provided the same effect as a dosage of 18 mg/kg ODN. This contrasts with free ODN (Fig. 8B) where 0.5 mg/kg provides no significant effect compared to HBS controls.

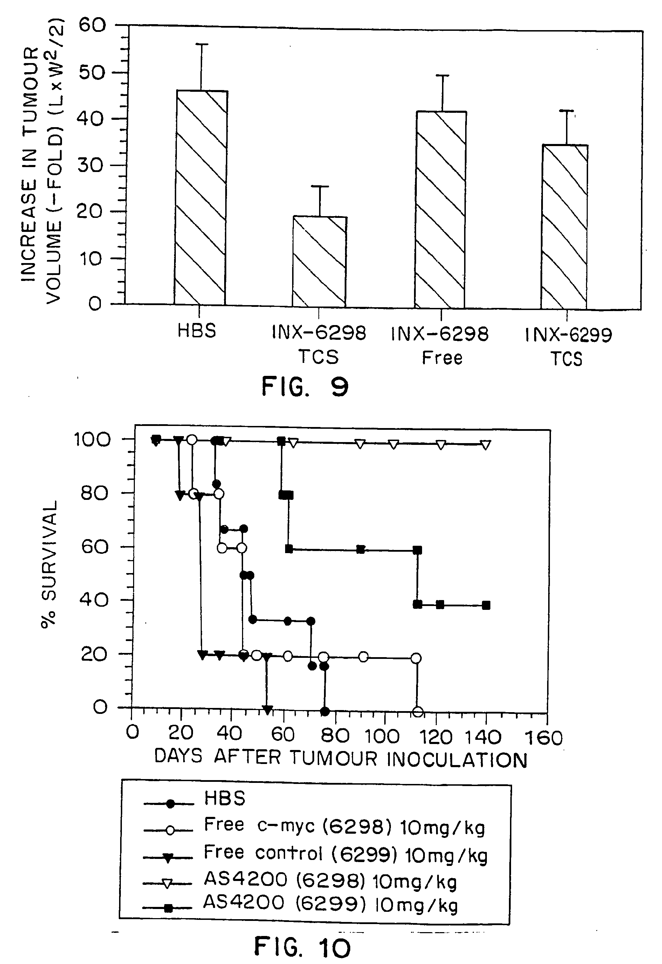

**Figure 9** demonstrates that INX-6298, an exclusively phosphodiester ODN, inhibits tumour growth when encapsulated in the TCS (AS4200), but not when delivered in the free form. Tumour volume was measured at Day 21 post tumour implantation.

Table 1 shows results demonstrating the efficacy of ODN formulations in NG Human Metastatic Melanoma model. MIce were injected with 2.5 X $10^6$ cells in hind leg muscle, to provide an ODN dose of 0.5 mg/mouse/day (-20 mg/kg/day) for 8 consecutive days (= 1 cycle). There was a 7 day interval between cycles, and a total of three cycles were performed. The ODN:lipid ratio = 0.20 w/w. Tumour weight inhibitions (TWI%) was calculated at the end of each cycle. Tumour growth delay (T-C) is the mediam time (in days) for treated and control tumors to reach the same size.

| TABLE 1 | | | | | | |
|---|---|---|---|---|---|---|
| Sample | TWI% 1 st Cycle | TWI% 2nd Cycle | TWI% 3rd Cycle | T-C (days) | % reduction in lung metastasis | % increase in life span |
| LR-3280 | 37 | 41 | 50 | 8 | 24 | 28 |
| INX-6295 | 39 | 43 | 49 | 8 | 28 | 30 |
| INX-6300 | 13 | 4 | 9 | 1 | 0 | 12 |

(continued)

| TABLE 1 | | | | | | |
|---|---|---|---|---|---|---|
| Sample | TWI% 1 st Cycle | TWI% 2nd Cycle | TWI% 3rd Cycle | T-C (days) | % reduction in lung metastasis | % increase in life span |
| INX-6295 AS4200 | 53 | 55 | 63 | 16 | 72 | 49 |
| INX-6300 AS4200 | 23 | 20 | 26 | 3 | 10 | 14 |

**Figure 10** is a survival curve of SCID-Rag2 mice carrying DoHH2 tumours treated every 2 days for 10 days beginning on day 4 after tumour inoculation with various formulations of c-myc or control ODN. Mice received an i.v. injection of 1 X $10^6$ Survival was monitored for 150 days. Each group consisted of 5-6 mice. Quite remarkably, an AS4200 formulation of PO-ODN 6298 essentially cures the tumour (n = 5-6 mice). The PS control (6299) AS4200 demonstrates significant survival enhancement over free ODN.

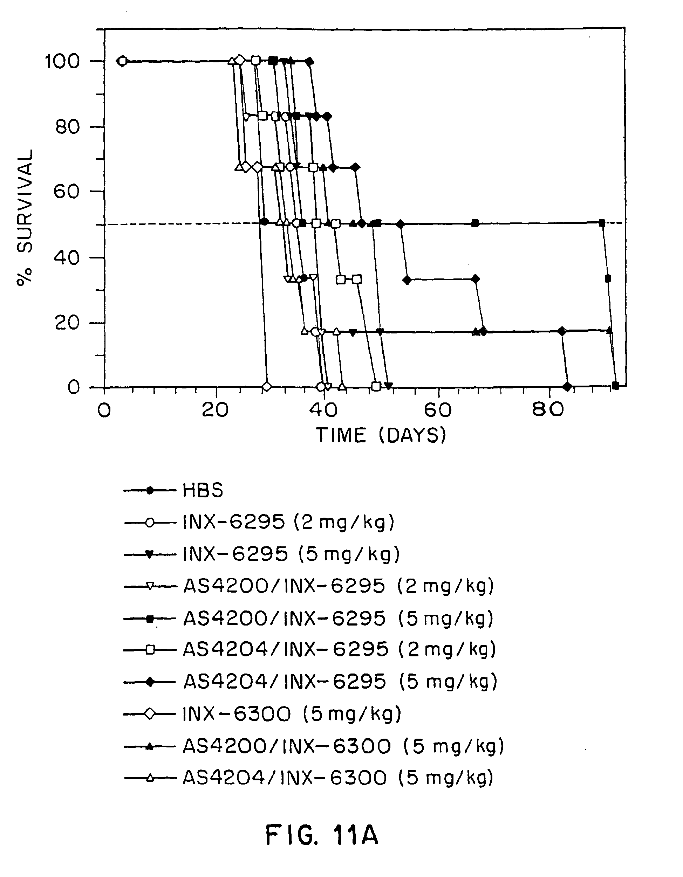

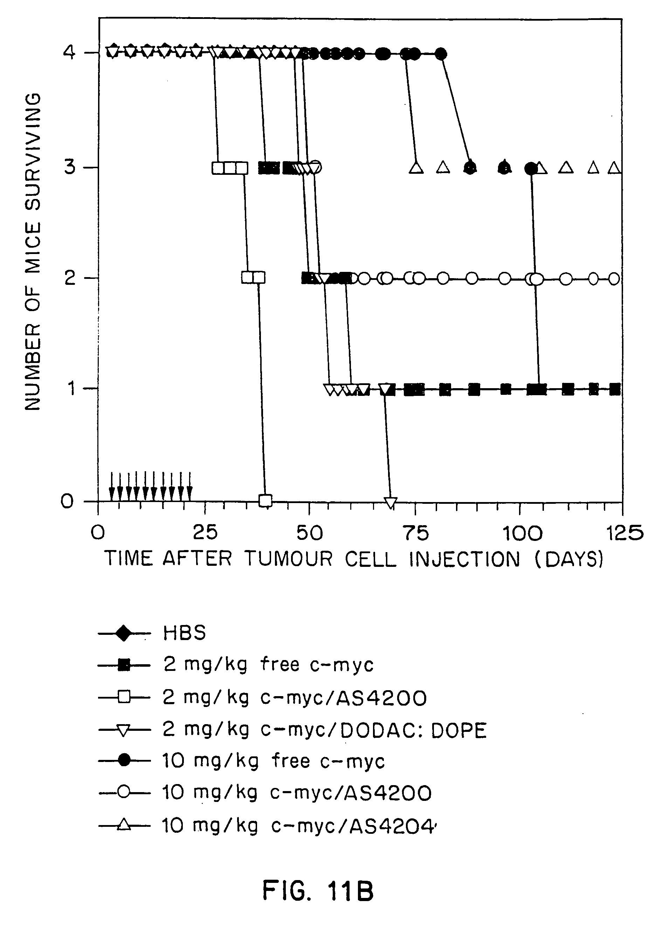

**Figure 11A** shows the dose response to free and AS4200 INX-6295 in i/v/ DoHH2 mice. A constant D/L ratio (high D/L, 0.20 initial) was employed for the AS4200 formulations. Dosing was every 2 days for 10 days. The doses of each agent were obtained through dilution in HBS, pH 7.6. Lipid doses varied (50 and 20 mg/kg). As shown, AS4200 INX-6295 treatment results in a much greater survival of DoHH2 mice compared to the free form of the drug. The free form of the drug does not provide a statistical improvement over HBS controls in this model, at this dosage (5 mg/kg). Additionally and quite remarkably, a significant, though smaller, benefit is derived from AS4200/INX-6300 at 5 mg/kg. INX-6300 does not carry ISS sequences, and its use was not expected to provide any survival advantage. (see below for further characterization of INX-6300). The survival advantage attributed to the AS4204 formulations is attributed to the fact that these mice are B-cell deficient, and thus do not generate a rapid clearance response. The B-cells are thus responsible for the clearance effect, but not necessarily for the treatment effect/survival advantage of treatment. In fact, the long circulating aspect of AS4204 formulation appears to enhance the survival advantage of treatment over AS4200. **Figure 11B** shows similar results at different dosage levels, i.e, lipid doses varied from 100 and 20 mg/kg. As shown, the treatment advantages were also found at higher (10 mg/kg) doses.

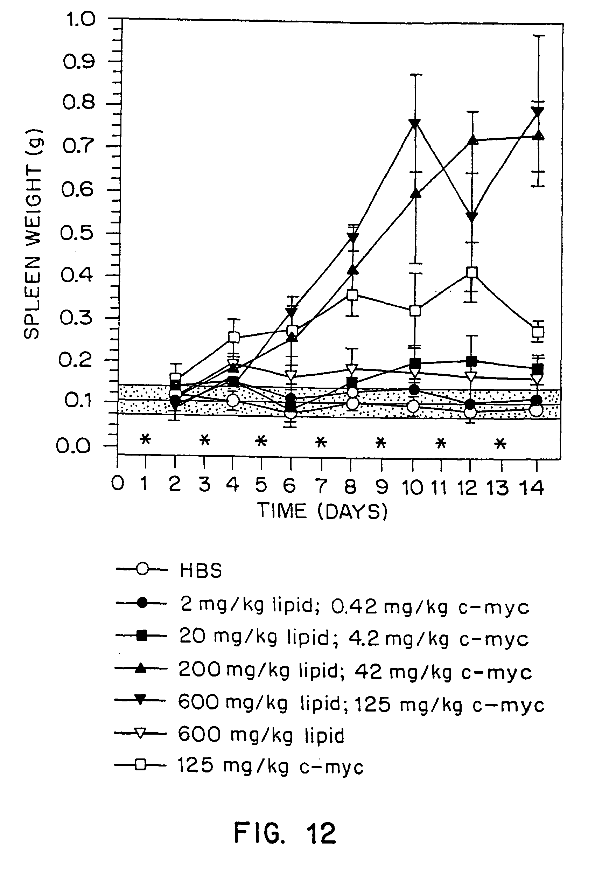

Some doses of LR-3280 (c-myc), administered i.v., were found to induces splenomegaly *in vivo* as reflected in the enlarged spleens of mice in response to some of the particles of the invention. **Figure 12** demonstrates that lipid itself (600 mg/kg) does not induce splenomegly, free c-myc (LR-3280) (125 mg/kg) induces a mild splenomegly, and AS4200 encapsulated LR-3280 induces splenomegaly at high doses (>200 mg/kg lipid and 42 mg/kg ODN) but not below 20 mg/kg lipid and 4.2 mg/kg ODN. The lipid encapsulated doses induce a significantly greater spleen enlargement than does free ODN.

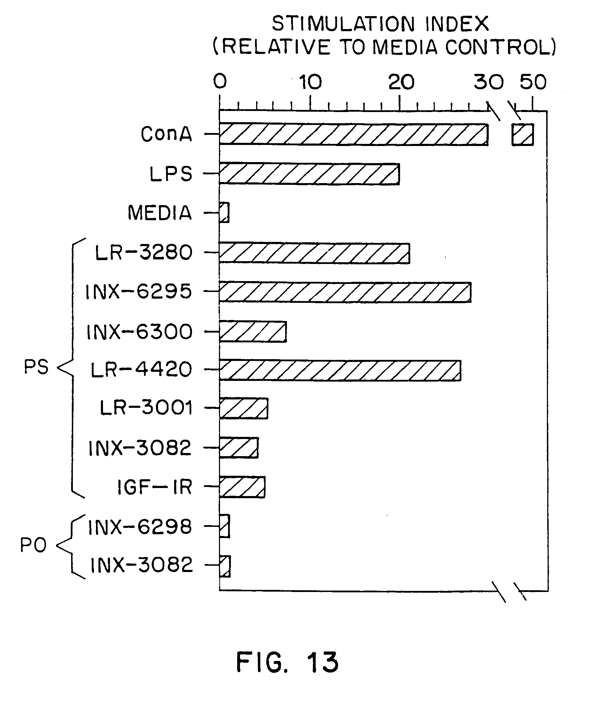

**Figure 13** shows the mitogenicity of various free ODN and controls towards *in vitro* splenocytes. All PS ODN demonstrate a background stimulation effect, but the greatest effect is found in the ISS containing sequences. This effect is equal to or greater than standard and well known mitogens LPS and ConA. PO-ODN show no effect, possibly because of degradation in the serum buffer. Not shown is results of methylated INX-6295 which demonstrated much reduced mitogenicity compared to unmethylated INX-6295, though its activity was not completely eliminated, being comparable to other PS ODN.

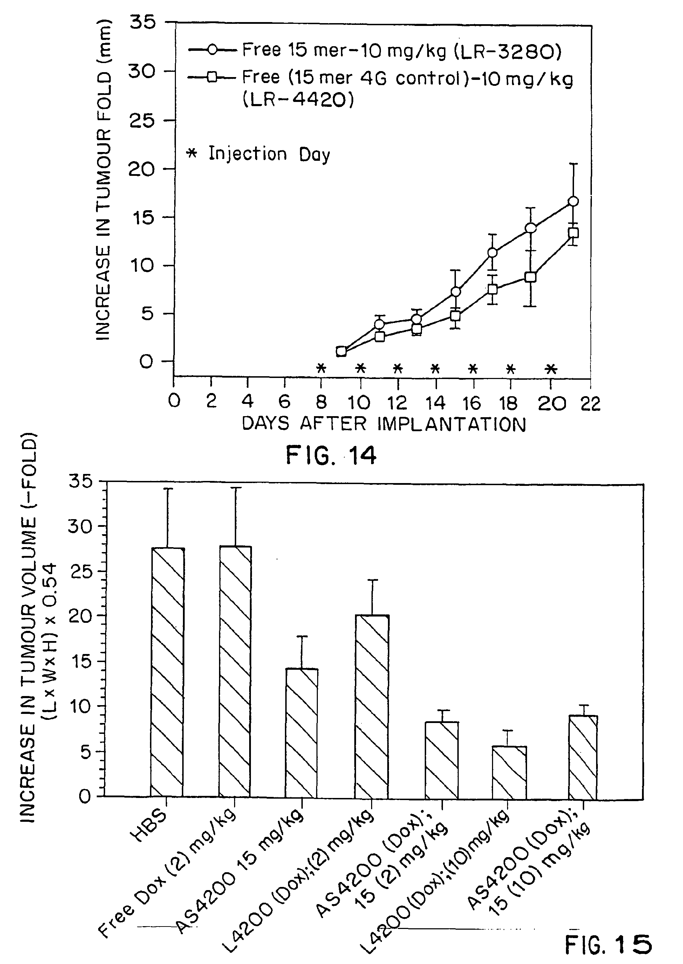

The mitogenicity of LR-4420 was further investigated because of its activity in the **Figure 13** results. As can be seen in **Figure 14**, a dose of 10 mg/kg, free LR-4420 has equal or improved tumour inhibition effects over free LR-3280. (See also Figure 7 for relative activity of free and AS4200 formulations of LR-3280).

Doxorubicin and c-myc ODN were coencapsulated in AS4200 liposomes. **Figure 15** summarizes the results. (Amounts of doxorubicin in "( )" in mg/kg. AS4200 includes ODN, L4200 is lipid-encapsulated doxorubicin with no ODN. In this figure, AS4200 contains INX-6295). Results show that at Day 21, AS4200 (15 mg/kg) co-encapsulated with doxorubicin (2 mg/kg) provides a surprising and statistically significant improvement over separately administered formulations. Further, increasing the doxorubicin in the AS4200 to 10 mg/kg does not improve the response, although encapsulated doxorubicin alone at 10 mg/kg provides the same response. These results indicate a complex interaction of cells and responses may be taking place in the combination therapy provided by the particles of this invention. It is possible that the increased dose of dox. may counteract the effect of the ODN.

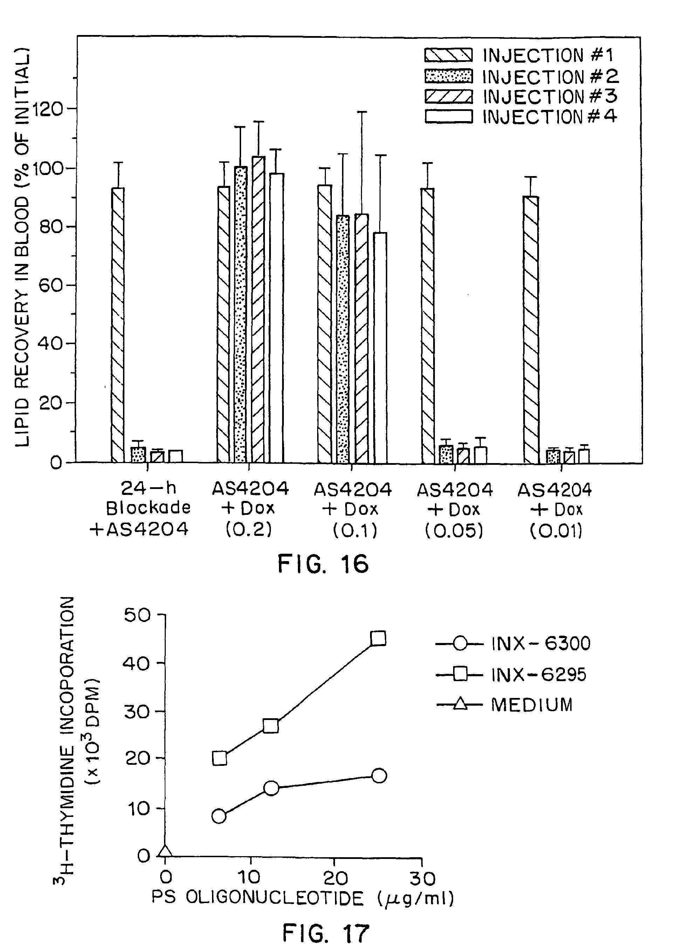

To determine if liver RES, in particular Kupffer cells, are involved in the clearance response, Kupffer cell inhibition was performed *via* RES blockade. This was accomplished by administering a low dose of encapsulated doxorubicin (sufficient to kill mature Kupffer cells) 24 hours prior to administering AS4204. Further, co-encapsulated doxorubicin in AS4204 at initial doxorubicin/lipid ratios of 0.2, 0.1, 0.05 and 0.01 were administered weekly to evaluate the corresponding *in vivo* response. Lipid recovery (% of initial) in the blood is plotted in **Figure 16.** It is evident that blockade with DSPC/Chol doxorubicin had no effect on inhibiting circulation elimination as less than 5% of the initially administered lipid was present in the circulation after the 2nd and subsequent injections. However, co-encapsulation of doxorubicin in AS4204 at initial ratios of 0.2 and 0.1 resulted in maintaining circulation levels of three subsequent injections to greater than 75% of the initially administered formulations. The threshold for inhibiting circulation elimination, therefore, appears to lie between 0.1 and 0.05. These results suggest Kupffer cells are not solely responsible for the clearance response; and that other cells which are disabled by co-administered dox. greater than 0.05 are largely responsible. These other cells may be B-cells or may be cells which activate B-cells; or they may be peripheral immune system cells such as tumour associated macrophages, etc.

Example 3

This series of examples characterizes some of the immune responses generated by the administration of lipid-nucleic acid particles of the invention. All methods and materials were identical to those of Example 1 & 2, with changes where indicated. These examples demonstrate unexpected qualities of SALP formulations which may be exploited for therapeutic benefit.

*Cell Lines and Mouse Strains.* YAC-1 cells were cultured in cRPMI (RPMI 1640,10% FCS, 50$\mu$M 2-mercaptoethanol, 2mM L-glutamine, 100U/ml steptomycin, 100$\mu$g/ml penicillin). All tissue culture media reagents were purchased from GIBCO BRL (Gaithersburg, MD, USA) and FALCON plasticware was purchased from Becton Dickinson (Franklin Lakes, NJ, USA). Female C57B1/6 mice were obtained from Harlan Sprague Dawley (Indianapolis, IN, USA). Female C57BL/6J-*Lystbg-J/+* (beige) mice were obtained from The Jackson Laboratory (Bar Harbor, ME, USA). It should be noted that when the beige mice were used, the wild-type C57B1/6J controls were also obtained from Jackson.

*Harvesting of Hepatic Mononuclear Cells (HMNCs).* Mice were euthanized by an overdose of anesthetic [3.2% (v/v) ketamine / 0.8% (v/v) xylazine]. The animal was then perfused via the left atrium with 6 mls of Hank's Balanced Salt Solution (HBSS) pre-warmed to 37°C followed by 6 mls of 0.25% collagenase IV (Sigma, St. Louis, MO, USA) in HBSS (also pre-warmed). Following a 15 minute digestion period, the liver was removed, briefly dispersed by hand in a 100 mm petri dish containing 5 ml of ice-cold RPMI 1640 with 5% FCS added (RPMI-5%) and transferred into a 50 ml conical tube on ice containing a total of 20 ml RPMI-5%. The liver was then dispersed mechanically by passing the digestion products through a 100 $\mu$m steel mesh. Hepatocytes were removed from the suspension by a 3-min centrifugation at 600 rpm. The remaining cells were pelleted by centrifugation at 1300 rpm for 5 min and washed once with HBSS. Hepatic mononuclear cells were isolated by resuspending the cell pellet in 30% Percoll (Amersham Pharmacia Biotech; Baie d'Urfe, PQ, Canada) in PBS and centrifuging at 2000 rpm for 10 minutes. The Percoll was carefully removed and the cell pellet was washed twice with 10 ml of HBSS and resuspended in a final volume of 2 ml cRPMI. Routinely, this process yielded 2-3 x $10^6$ mononuclear cells from the liver of an 8-10 week old C57B1/6 mouse.

*Chromium release assay.* To measure NK activity in the mononuclear cell preparations, cell suspensions were tested for their ability to lyse $^{51}$Cr labeled NK- target cells (YAC-1) as described by Bramson et al. (1996). Briefly, YAC-1 cells were labeled with $^{51}$Cr by incubating $10^6$ cells in 50$\mu$Ci of $^{51}$Cr (NEN Life Science Products; Boston, MA, USA) for 1 hour at 37 °C. The labeled YAC-1 cells were resuspended in cRPMI at a concentration of $10^6$ cells/ml and 50 $\mu$l aliquots of the YAC-1 suspension were mixed with varying numbers of peritoneal exudate cells in U-bottomed 96-well plates to yield effector: target ratios of 90:1, 30:1, and 10:1. The plates were incubated at 37°C, 5%CO$_2$ for 4-6 hours. Following the incubation period, 100$\mu$l of the culture supernatant was removed from each well for scintillation counting.

*FACS Analysis.* The surface expression of NK 1.1 and T cell receptor (TCR) $\beta$-chain on mononuclear cell preparations was determined by 2-color flow cytometry analysis using the following antibodies: PE-conjugated anti-NK1.1 (clone PK136), FITC-conjugated anti-TCR$\beta$ (clone H57-597), PE-conjugated mouse IgG$_{2a}$, $\kappa$ isotype control (clone G155-178) and FITC-conjugated Hamster IgG, group 2, $\lambda$ isotype control (clone Ha4/8). All antibodies were obtained from Pharmingen (Mississauga, ON). The percentage of single and double positive cells in the population was calculated using the CellQuest software package (Becton-Dickinson, San Jose, CA). The absolute number of NK and NKT cells was determined by subtracting the percentage of non-specifically stained cells from the percentage of positively stained cells and multiplying by the total cell number.

*Statistical Analysis.* All data are expressed as mean $\pm$ SEM and compared using, two-tailed Student's t-test. The statistics were calculated using Statview 512+ for Macintosh (Abacus Concepts, Inc., Berkeley, CA).

**Figure 17** demonstrates that in a mitogenicity assay of INX-6295 (CpG containing) and INX-6300 (CpG absent), INX-6295 is at least 4 times more mitogenic. Nonetheless, INX-6300 did possess stimulatory activity compared to medium alone, consistent with reports that the PS backbone itself is a mitogenic agent. Naïve splenocytes were incubated in vitro with graded amounts of free PS ODN (6.25 $\mu$g/ml to 25 $\mu$g/ml). The cultures were pulsed with $^3$H-thymidine and harvested 72 h later. Each point represents the mean $^3$H-thymidine incorporation $\pm$ SEM of 3 separate cultures. This figure represents 1 of 2 experiments performed in triplicate Closed squares, free INX-6295; closed circles, free INX-6300.

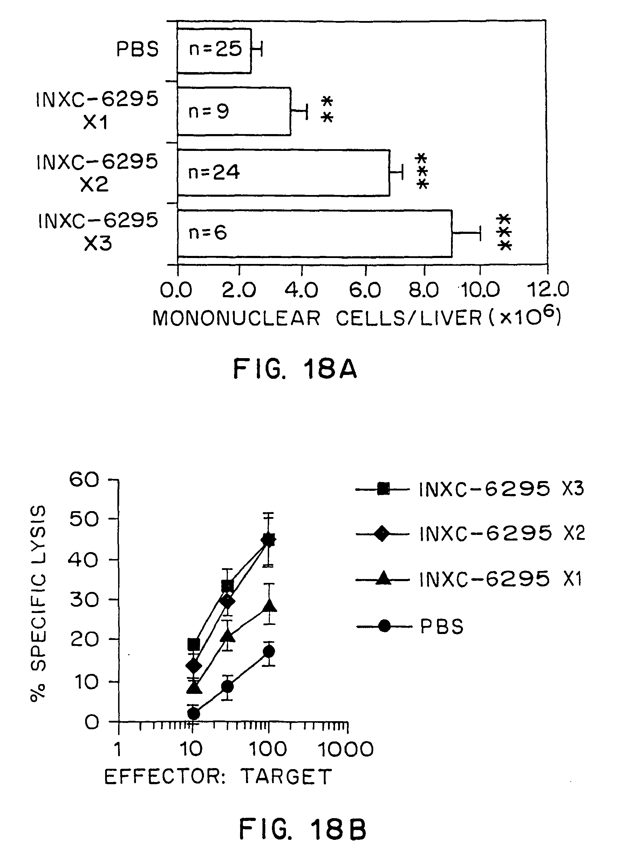

C57B1/6 mice received one (6295 X 1), two (6295 X 2), or three (6295 x 3) intravenous injections of INX-6295/SALP (15 mg/kg ODN; 48 h between injections) or 3 intravenous injections of PBS (48 h between injections). Hepatic mononuclear cells (HMNC) were harvested 24 h following the final injection. **Figure 18A** shows the total number of HMNC. Each bar represents the mean HMNC + SEM of 6 - 25 mice. **Figure 18B** shows the lytic activity of HMNC against YAC-1 cells. Each point represents the mean % specific lysis $\pm$ SEM of 3 separate HMNC populations. This figure represents 1 of two experiments performed in triplicate. Squares, 3 injections of INX-6295/SALP; diamonds, 2 injections of INX-6295/SALP; triangles, 1 injection of INX-6295/SALP; circles, 3 injections of PBS. ***, p<0.0001; **, p<0.01. As reflected in **Figures 18A,** three repeated injections of INXC-6295 result in a linear increase in Mononuclear Cells and Natural Killer Activity in the Liver. Splenomegaly was observed following the second and third administrations of INXC-6295 as determined by spleen weight, however, there was no increase in splenocyte number (data not shown), thus indicating that increases in cell number were not responsible for enlargement. **Figure 18B** shows that cytolytic activity of HMNC populations, as measured by YAC-1 chromium release assays was increased following INXC-6295 treatment. The similarity of slope between the lysis curves of the HMNC populations harvested following the second and third administrations of INXC-6295 suggests that the same effector cells are present in both samples. It was also observed that NK activity within the spleen increased following subsequent administration of INXC-6295, albeit to a lesser extent than the HMNCs.

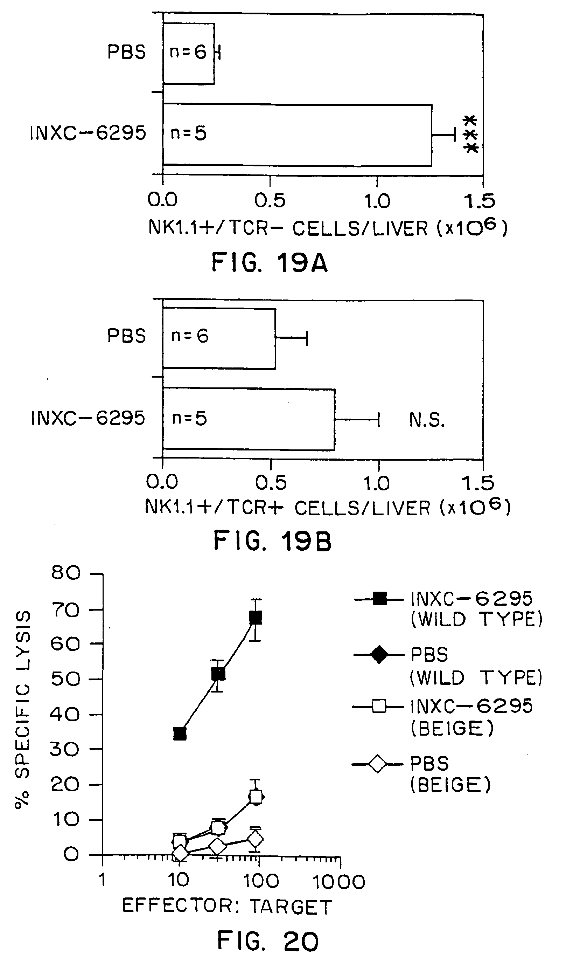

Groups of 3 C57B1/6 mice received 2 intravenous injections of INX-6295/SALP (15 mg/kg ODN; 48 h between injections) or PBS (48 h between injections). Hepatic mononuclear cells (HMNC) were harvested 24 h following the final injection. Equal numbers of cells were pooled from triplicate samples and surface expression of NK1.1 and TCR β chain was measured by flow cytometry. **Figure 19A** shows the total number of NK1.1+/TCR- cells in the HMNC pool. Each bar represents the mean cell number + SEM of 5-6 experiments. **Figure 19B** shows the total number of NK1.1+/TCR+ cells in the HMNC pool. Each bar represents the mean cell number + SEM of 5-6 experiments. ***, p<0.0001; N.S., not significant. As shown, there is an increase in NK1.1+/TCR- cells in the liver following INX-6295/SALP treatment. Since the YAC-1 cells are sensitive to both NK and NKT mediated lysis, it was not clear from the chromium release assays which population was responsible. There was no significant increase in NKT cells following INXC-6295 treatment. In contrast there was a 5-fold increase in hepatic NK cells following INXC-6295 treatment. These results strongly suggest that the cell population responsible for the increased lytic activity observed following administration of INXC-6295 was the NK cell.

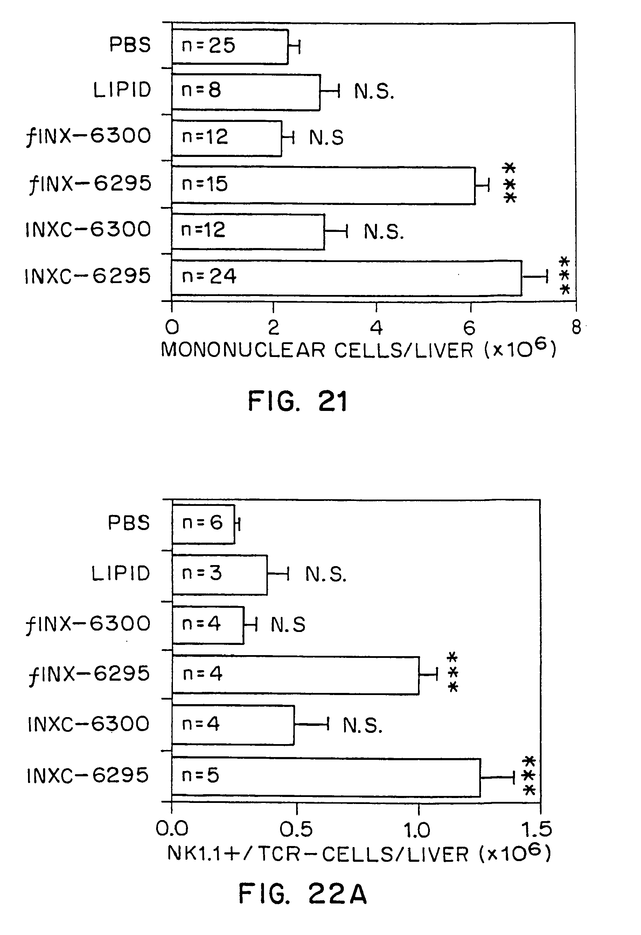

Groups of 3 C57B1/6J mice (wild type) and 3 C57BL/6J-Lyst[bg-J]/+ (beige) received 2 intravenous injections of INX-6295/SALP (15 mg/kg ODN; 48 h between injections) or PBS (48 h between injections). Hepatic mononuclear cells (HMNC) were harvested 24 h following the final injection. HMNC were tested for lytic activity against YAC-1 cells. The results are shown in **Figure 21,** where each point represents the mean % specific lysis $\pm$ SEM of 3 separate HMNC populations. Closed squares, 2 injections of INX-6295/SALP, wild type mice; closed diamonds, 2 injections of PBS, wild type mice; open squares, 2 injections of INX-6295/SALP, beige mice; open diamonds, 2 injections of PBS, beige mice. As shown in **Figure 21,** there is a lack of cytolytic activity in the HMNC from *beige* mice (NK cell and B-cell deficient) following INX-6295/SALP treatment. Whereas INXC-6295 induces a strong lytic activity in wild type mice, only weak lytic activity was observed in *beige* mice given the same treatment. This suggests that NK cells are specifically activated by the particles of the invention. The weak lytic activity in *beige* mice may still be attributed to inefficient suppression of NK activity in the mouse, or it may be evidence of a small component of NKT cell activity. *Beige* mice bearing tumours also demonstrate no treatment response or efficacy of INXC-6295, thus suggesting that the NK cell and B-cell responses are key cells to be activated by the particles of the invention.

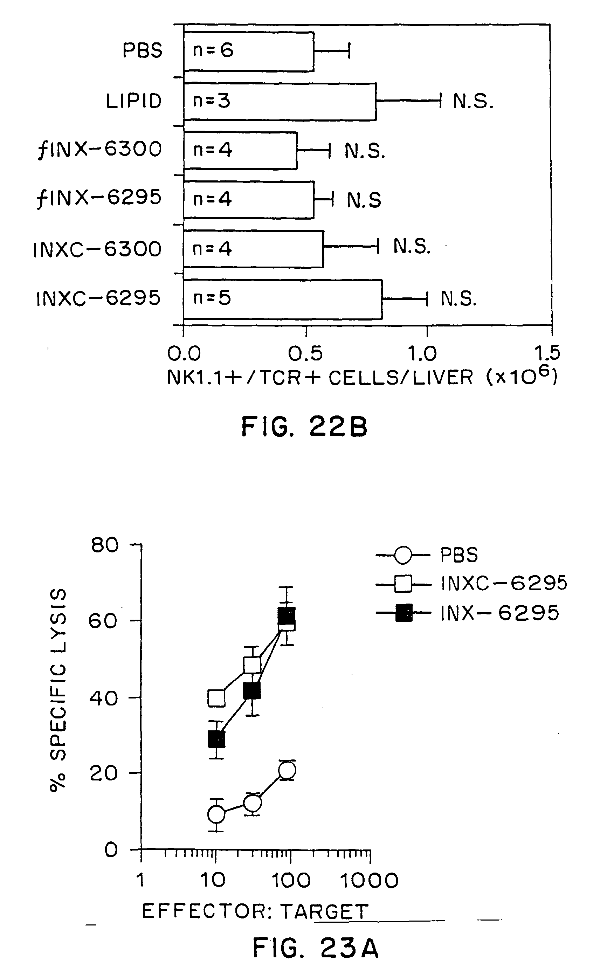

C57B1/6 mice received 2 intravenous injections of INX-6295/SALP, INX-6300/SALP, free INX-6295, free INX-6300 (15mg/kg ODN; 48 h between injections), lipid alone or PBS (48h between injections). HMNC were harvested 24 h following the final injection. The results are shown in **Figure 22,** where each bar represents the mean HMNC + SEM of 8 - 25 mice. ***, p=0.0001; N.S., non-significant. As shown, there is an increase in HMNC following administration of free and encapsulated PS ODN. Administration of free INX-6295 produced increases in HMNC similar to that observed for the AS4200 formulation of INX-6295. However, INX-6300 in AS4200 only generated a minor expansion of HMNCs compared to PBS, while free INX-6300 did not induce any increase.

Groups of 3 C57B1/6 mice received 2 intravenous injections of INX-6295/SALP, INX-6300/SALP, free INX-6295, free INX-6300 (15 mg/kg ODN; 48 h between injections), lipid alone or PBS (48 h between injections). HMNC were harvested 24 h following the final injection. Equal numbers of cells were pooled from triplicate samples and surface expression of NK1.1 and the TCR β chain was determined by flow cytometry. **Figure 22A** shows the total number of NK1.1+/TCR- cells in the HMNC pool. Each bar represents the mean cell number + SEM of 3-6 experiments. **Figure 22B** shows the total number of NK1.1+/TCR+ cells in the HMNC pool. Each bar represents the mean cell number + SEM of 3-6 experiments. ***, p<0.0001; N.S., non-significant. As shown, there is an increase in NK1.1+/TCR- cells in the liver following SALP treatment. NK cell expansion is also found following treatment with free or encapsulated INX-6295, and to a reduced (non-significant) degree with INXC-6300. None of the treatments produced significant changes in the NKT compartment.

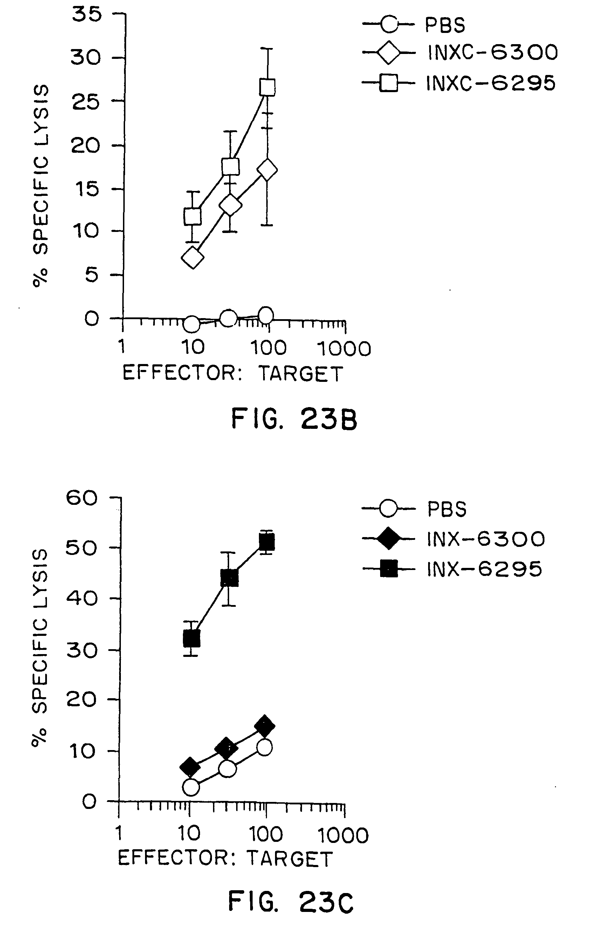

Groups of 3 C57B1/6 mice received 2 intravenous injections of INX-6295/SALP, INX-6300/SALP, free INX-6295, free INX-6300 (15 mg/kg ODN; 48 h between injections) or PBS (48 h between injections) on days 0 and 2. Hepatic mononuclear cells (HMNC) were harvested on day 3, 24 h following the final injection. HMNC were tested for lytic activity against YAC-1 cells. The results are shown in **Figures 23 A-C**, where each point represents the mean % specific lysis ± SEM of 3 separate HMNC populations. Each graph is representative of 2-3 experiments performed in triplicate. Closed squares, 2 injections of free INX-6295; closed diamonds, 2 injections of free INX-6300; open squares, 2 injections of INX-6295/SALP; open diamonds, 2 injections of INX-6300/SALP; open circles, 2 injections of PBS. As shown, there is activation of Natural Killer cells within the HMNC population following administration of free and encapsulated PS ODN. Intravenous SALP administration can activate liver NK cells in the absence of an ISS motif. **Figure 23A** shows that both free and encapsulated INX-6295 promote similar activation of liver NK cells. Surprisingly, INX-6300/SALP produced alsmost as much lytic activity as INXC-6295, indicating that SALP (or AS4200) formulations of a non-ISS sequence can produce ISS type responses. This establishes a new class of ISS motifs, which do not activate immune responses in the free form, but instead are dependent upon lipid-particle encapsulation to produce an immune response.

Taken together, the results of this Example show that immune activation by INX-6300/SALP, but not with either the ODN or lipid on their own, may represent an additional pathway for immunostimulation independent of ISS motifs or double stranded nucleic acid. It should be noted, however, that while the NK cells stimulated by INX-6300/SALP (lacks an ISS motif) exhibited lytic activity similar to those stimulated by INX-6295/SALP (contains an ISS motif), expansion of the NK cells was only observed when the ODN payload contained an ISS motif. This suggest that NK cells may require multiple signals for activation of lytic activity and proliferation or that the signal elicited by INX-6300/SALP is strong enough to activate cytotoxicity but too weak to promote expansion. Modifications to the SALP formulation using stimulatory lipids such as $\alpha$-galactose ceramide may provide the additional stimulus to promote expansion and activation of liver NK cells and possibly NKT cells.

Example 4

This series of examples illustrates the ability of certain cationic lipid:DNA complexes (non-encapsulated systems or lipoplexes) to generate immune responses, thus providing a functional adjuvant for cancer gene therapies.

*Reagents.* DODAC (N,N-dioleyl-N,N-dimethylammonium chloride) and DOTMA (N-[1-(2,3-dioleyloxy)propyl]-N,N,N-trimethylammonium chloride) were prepared by Dr. Steven Ansell (Inex Pharmaceuticals; Vancouver, BC, Canada). DOTAP (N-[1-(2,3-dioleoyloxy)propyl]-N,N,N-trimethylammonium chloride) and DOPE (1,2-dioleoyl-sn-glycero-3-phosphoethanolamine) were obtained from Avanti Polar Lipids (Alabaster, AL, USA). Actinomycin D and N-(1-Napthyl)ethylenediamine, sulfanilamide were purchased from Sigma (St.Louis, MO, USA).

*Cell culture.* MCA207 (murine fibrosarcoma) (provided by S. Rosenberg, National Cancer Institute, Frederick/Bethesda, MD), SKOV-3 (human ovarian carcinoma, ATCC#HTB-77), LS180 (human colorectal carcinoma), and WEHI 13VAR (ATCC#CRL-2148) were cultured in cRPMI [RPMI 1640,10% FCS, 50$\mu$M 2-mercaptoethanol, 2mM L-glutamine, 100U/ml steptomycin, 100$\mu$g/ml penicillin]. All tissue culture media reagents were purchased from GIBCO BRL (Gaithersburg, MD, USA) and FALCON plasticware was purchased from Becton Dickinson (Franklin Lakes, NJ, USA).

*Preparation of LUVs.* Lipid films were prepared by lyophilization of lipid solutions composed of 10 mg/ml lipid in 100% ethanol. The lipids were resuspended in water at a final concentration of 40mM lipid. The solubilized liposomes were then extruded 10 times through a 100nm carbonate membrane to generate Large Unilamellar Vesicles (LUVs) and stored at 4˚C. The LUVs used in these studies were composed of a 1:1 molar ratio of cationic lipid (DODAC, DOTMA or DOTAP) and DOPE.

*Lipoplex formation* LUV/DNA complexes (which are "non-fully encapsulated systems" for the purposes of this specification) were prepared at a charge ratio of +3. A solution was prepared containing pCMVluc18 at a final concentration of 500$\mu$g/ml in 5% glucose. The DNA solution was added dropwise to a solution containing 9.0mM DODAC:DOPE (1:1) LUVs in 5% glucose while vortexing. The complexes were then incubated for 30 minutes at 4˚C. Lipoplexes were prepared fresh prior to each use.

*Luciferase and Protein Assays.* Luciferase assays were performed using the Luciferase Assay System kit (Promega; Madison, WI, USA) as described previously (12). Cellular lysates were assayed for protein content using the bicinchoninic acid colorimetric method (Pierce Chemical Co.; Rockford, IL, USA) according to the manufacturer's protocol.

*Murine Peritonitis.* Female C57B1/6 mice (Harlan Sprague Dawley; Indianopolis, IN, USA) received an intra-peritoneal injection of LUVs or lipoplexes in 200$\mu$l of 5% glucose (lipid dose = 60mg/kg). At specified time points, the mice were euthanized by asphyxiation with $CO_2$ and peritoneal exudate cells were recovered by lavage with 5 mls of ice-cold Hanks Balanced Salt Solution (HBSS). The concentration of cells in the lavage was quantified using a cell counter (Coulter Diagnostics; Hialeah, Fla., USA) and the cells were washed twice with HBSS. After the final wash, the cell pellet was resuspended in cRPMI.

*NK assay.* To measure NK activity, peritoneal exudate cells were tested for their ability to lyse $^{51}$Cr labeled YAC-1 cells as described in Bramson et al. (13). One lytic unit (LU) is equal to the number of exudate cells required to produce 30% specific lysis of 5000 YAC-1 cells.

*TNF-α bioassay.* The TNF-α bioassay was carried out as described by Khabar et al. (14) with the following modification: At the end of the assay period 100μl of supernatant was removed from each well, 20μl of MTS solution (CellTiter 96 Aqueous Non-radioactive Cell Proliferation Assay; Promega, WI, USA) was added to each well and the plates were incubated a further 1.5 hours. The absorbance of the solution in each well was measured at 490nm. The concentration of TNF-α in the experimental wells was calculated by comparison to recombinant standards. Routinely, this assay was sensitive to 15pg/ml recombinant standard.

*Cytokine ELISAs.* IFNγ **(**Endogen; Woburn, MA, USA) and IL-12p70 (Pharmingen; San Diego, CA, USA) levels were measured using a specific ELISA as described by the manufacturer.

*Nitric Oxide (NO) release assay.* Aliquots of $5 \times 10^5$ peritoneal exudate cells were transferred to 24 well plates. The cells were then incubated for 24 hours in medium +/-10ng/ml LPS in a total volume of 1ml. Following the incubation period, the concentration of nitrates in the supernatant was determined using the Griess Assay. A 100μl aliquot of the culture medium was mixed with 100μl of Griess Reagent [equal volumes of Griess Reagent A (0.1% N-(1-Napthyl) ethylenediamine) and Griess Reagent B (1% Sulfanilamide in 5% Phosphoric Acid)] in a flat-bottomed 96-well plate. The absorbance of each well was then measured at 570nm. Nitrate concentration was determined using a standard curve ranging from 1mM to 1.6μM.

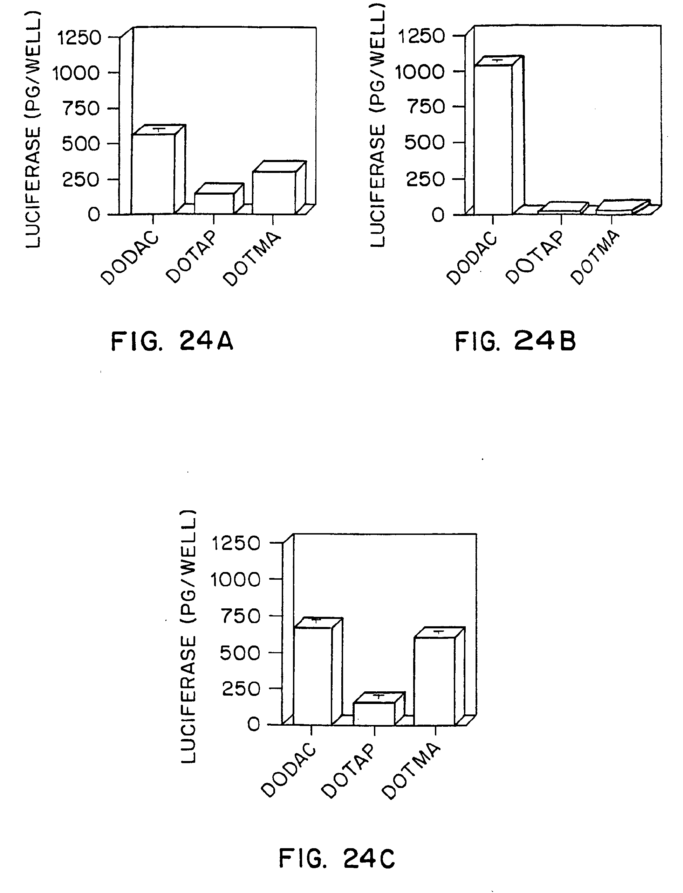

Cells were incubated overnight with 0.25μg/ml DNA complexed to 1 00nm LUVs composed of a 1:1 molar ratio of cationic lipid and DOPE. The cells were then lysed and assayed for luciferase expression as described in the "Materials and Methods" section. The results are shown in Figures 24 A-C. Three tumor cell lines were used in this experiment: murine fibrosarcoma MCA207 (Fig. 24A), human ovarian carcinoma SKOV-3 (Fig. 24B), and human colorectal carcinoma LS 180 (Fig. 24C). This data reflects 1 of 3 individual experiments. Each bar represents the mean RLU of 4 replicate transfections + s.e.m. The transfection profiles of lipoplexes containing either DODAC, DOTAP or DOTMA illustrate that different cationic lipids have different transfection abilities, and that different tumour cell lines respond differently to them. This suggests that particles of the invention may employ different cationic lipids depending on the indication (tumour type) which is being treated.

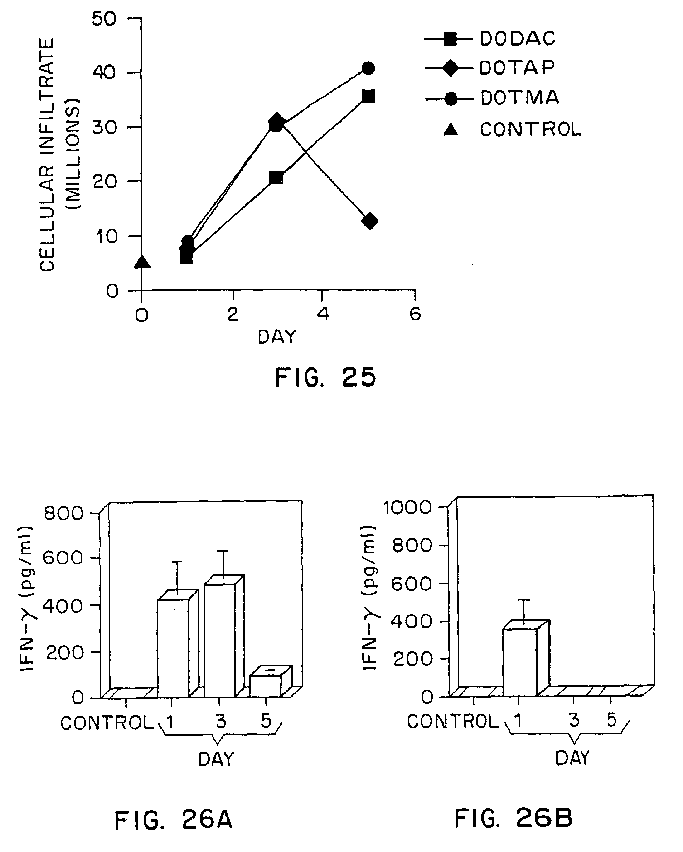

C57B1/6 mice were inoculated with 50μg of DNA complexed with LUVs composed of a 1:1 molar ratio of cationic lipid and DOPE. Peritoneal exudate cells were harvested on days 0, 1, 3, and 5. The results are shown in **Figure 25**, which represents the results of two independent experiments with 4-6 animals per group. Each point represents the mean cellular infiltrate in the lavages of 8-12 mice $\pm$ s.e.m. As shown, the cellular rate in the peritoneum increases following lipoplex administration, but DOTAP lipoplexes resolved to near normal levels by day 5.

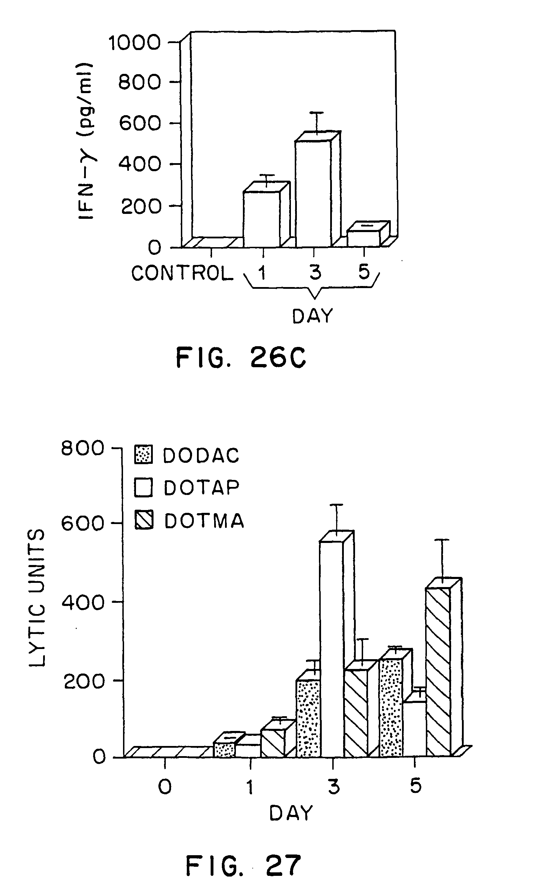

C57B1/6 mice were inoculated with 50μg of DNA complexed with LUVs composed of a 1:1 molar ratio of cationic lipid and DOPE. The peritoneal exudates were harvested by lavage on days 1, 3 and 5. **Figures 26A-C** show results for two independent experiments with 4-6 animals per group using DODAC (Fig. 26A), DOTAP (Fig. 26B) and DOTMA (Fig. 26C) as the cationic lipid. Each point represents the mean IFN-γ concentration in the lavages of 8-12 mice + s.e.m. As shown, cytokine IFN-γ levels responded differently to treatment with different cationic lipids. There was no detectable response for TNF-α or L-12 in these assays.

C57B1/6 mice were inoculated with 50μg of DNA complexed with LUVs composed of a 1:1 molar ratio of cationic lipid and DOPE. The peritoneal exudates were harvested by lavage on days 1, 3 and 5 and tested for cytotoxicity on $^{51}$Cr labeled YAC-1 cells. **Figure 27** shows the results of one of two independent experiments with 4-6 animals per group. Each point represents the mean lytic units within the lavages of 4-6 mice $\pm$ s.e.m. As shown, lipoplex induced activation ofNK cells parallels the accumulation of cellular infiltrate in these experiments. DODAC and DOTMA lipoplexes elicited a progressive increase in NK activity over a period of 5 days while the DOTAP lipoplexes induced NK activity which peaked at day 3 and remained substantially elevated at day 5. There is also an increase in activated macrophages within the peritoneal cavity over the course of the inflammatory response (results not shown).

Taken together, these results suggest that since inflammatory signals are required to achieve proper maturation and function of dendritic cells, the inflammatory response which follows lipoplex administration may reverse the effect of tumour derived cytokines. Further, since it was observed that lipoplex administration leads to increased local NK activity and NK cells have been shown to spontaneously lyse tumour cells, that the combined effects of lipoplex administration to a tumour microenvironment are likely to cause favourable treatment responses.

Example 5

This series of experiments was designed to investigate the induction f serum cytokines following administration of lipid-encapsulated ISS oligodeoxynucleotides.

*Materials and Methods*

Distearoylphosphatidylcholine (DSPC) and 1,2-dioleoyl-3-N,N-dimethylammonium-propane (DODAP) was purchased from Avanti Polar Lipids (Alabaster, AL, USA) while cholesterol was from Sigma (St. Louis, MO, USA). 1-O-(2'-(ω-methoxypolyethylene-glycol)succinoyl-2-N-myristoylsphingosine (PEG-CerC$_{14}$) was synthesized by Dr. Zhao Wang (Inex Pharmaceuticals Corp.). The ODN sequences used include the 15 mer c-myc ODN complementary to the initiation codon region of the human/mouse c-myc proto-oncogene mRNA (5'-AACGTTGAGGGGCAT-3') (SEQ ID No. 5), a 16 mer version of the same ODN (5'-TAACGTTGAGGGGCAT-3') (SEQ ID No. 4), and the ICAM-1 ODN (ISIS 3082) complementary to the 3' untranslated region of ICAM-1 mRNA(5'-TGCATCCCCCAGGCCACCAT-3') (SEQ ID No. 2). The c-myc ODNs were from Lynx Therapeutics (Hayward, CA, USA) while ISIS 3082 was purchased Boston Biosystems, Inc (Bedford, MA, USA). Female, 6 week old ICR mice were obtained from Harlan Sprague Dawley (Indianapolis, IN, USA) and were quarantined for at least one week prior to use.

*SALP.* SALP composed of DSPC:cholesterol:DODAP:PEG-CerC14 (20:45:25:10, molar ratio) and encapsulated PS ODN were prepared as previously described (Semple et al., 1999). For PS ODN, 300 mM citrate buffer was used to dissolve the ODN, whereas 20 mM citrate, pH 4.0 was used for PO ODN-containing SALP. Briefly, the lipid mixture dissolved in ethanol was added to the ODN (3.33 mg/ml) citrate buffer (40 % final ethanol concentration). The resulting vesicle mixture was freeze-thawed 5 times and extruded through 2 stacked 100 nm pore sized filters using an extruder (Northern Lipids, Van, BC, Can.). The vesicles were dialyzed for 2 h against citrate buffer to remove the ethanol then overnight in 500-fold volume of HBS (150 mM NaCl, 20 mM HEPES, pH 7.5) to neutralize the DODAP on the external monolayer. Non-encapsulated ODN was removed from the preparation by anion exchange chromatography using DEAE-sepharose CL-6B. The ODN to lipid ratio was calculated based on ODN quantification by A260 and lipid content by a phosphate assay (Fiske & Subbarow, 1925) assuming that the lipid mixture consisted of 20 mole percent DSPC. As the phosphate on the ODN backbone would interfere with the lipid analysis samples were subjected to a Bligh & Dyer (1959) followed by 3 water-methanol washes to remove the ODN. The ODN to lipid ratio was typically 0.15 - 0.20 (wt/wt). Vesicle sizes as determined by quasi-elastic light scattering using a NICOMP Submicron particle sizer (Model 370) were approximately 120 nm.

*Serum isolation.* ICR mice (7 week old at the start of the experiment) were injected intravenously with 0.2 ml of sample in HBS. At various times, the mice were killed by terminal dose of anesthetic (3.2 % (v/v) ketamine/ 0.8 % (v/v) xylazine) and blood collected Vacutainer tubes containing EDTA. The blood was centrifuged (2000 x g for 10 min at 4˚C) to pellet the blood cells and the serum isolated and frozen at -20˚C until assayed.

*ELISA.* Serum contents of IL-2, IL-4, IL-10, IL-12, IFN-γ, MCP-1 and TNF-α were determined using commercial ELISA kits (PharMingen, San Diego, CA, USA).

The immune stimulatory CpG ODN used in this example is an antisense ODN designed to be complementary to the initiation codon region of the murine and human c-myc proto-oncogene. Both the 15 mer and 16 mer version of this ODN have shown activity against a variety of human and murine tumors *in vitro* and *in vivo* (Leonetti et al., 1996; Citro et al., 1998; Harasym et al., manuscript in preparation). However, both ODNs (the 16 mer being identical to the 15 mer except for an extra thymidine at the 5' end) contain a known stimulatory CpG motif, 5'-(T)AACGTT-3', (Ballas et al., 1996). The control ODN sequence used in this study is ISIS 3082, a 20 mer antisense ODN complementary to the 3' untranslated region of murine ICAM-1 mRNA. In contrast to the c-myc ODN, ISIS 3082 does not contain CpG motifs and is not immunogenic *in vitro* (Boggs et al., 1997).

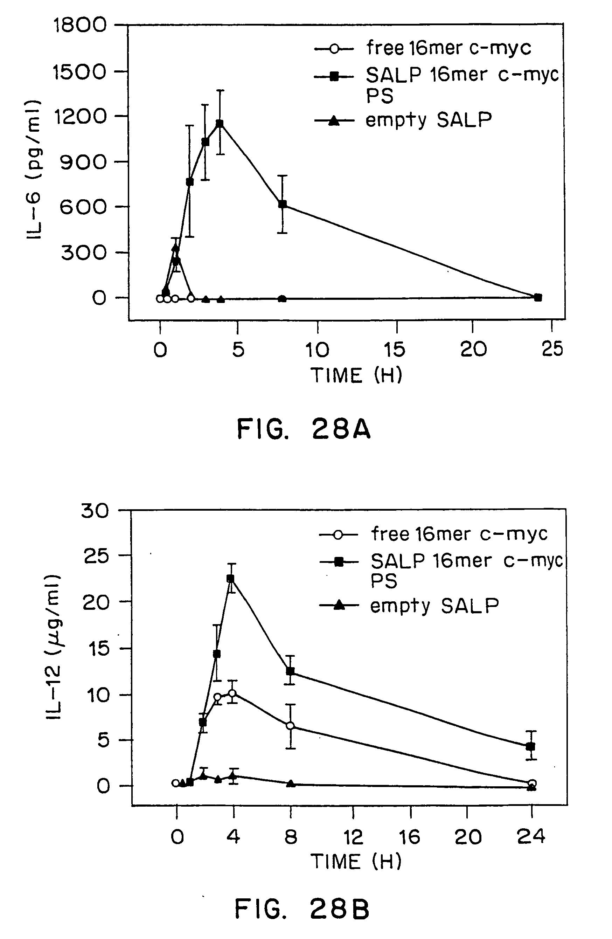

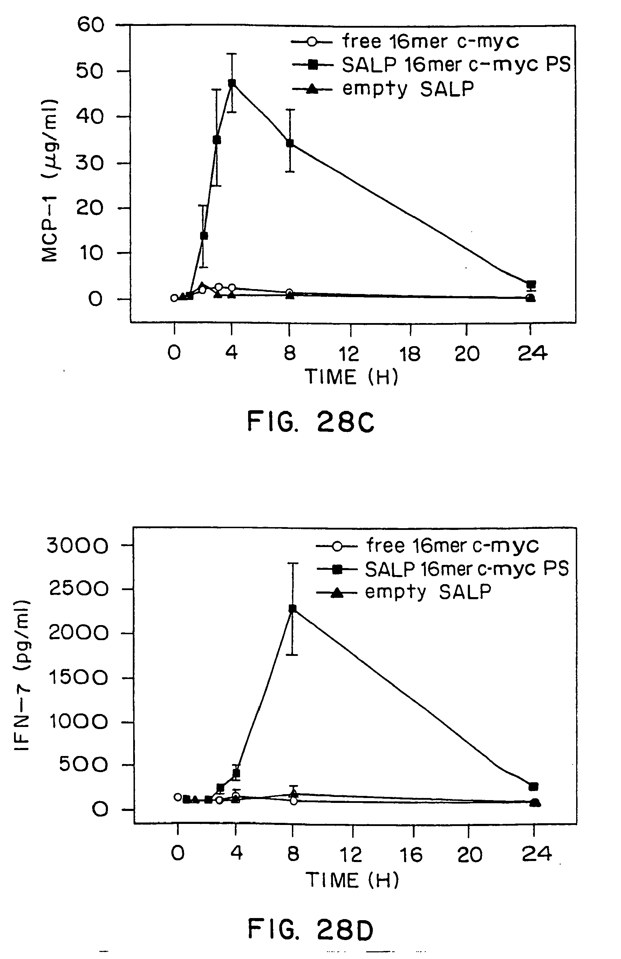

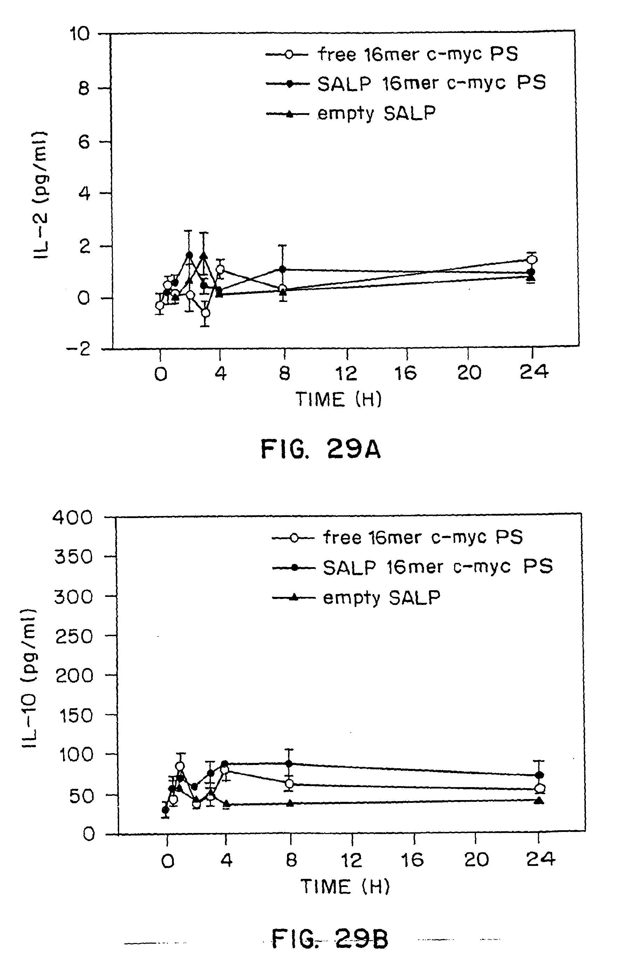

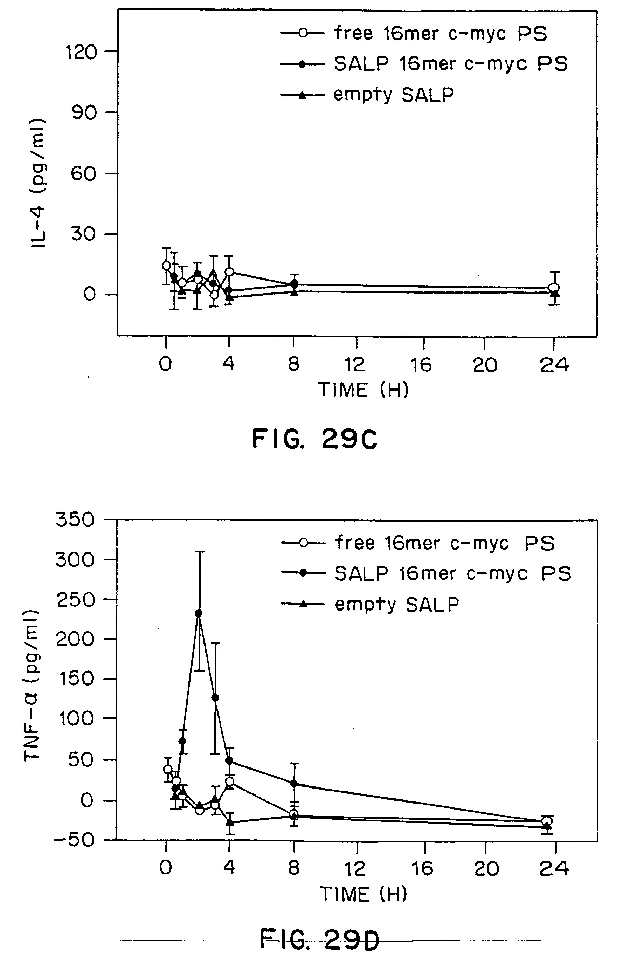

An immune response to free PS ODN has been previously observed in terms of increased serum cytokine levels. ICR mice treated with an i.p. injection of free PS ODN (50 mg/kg) have elevated levels of IL-12, IL-6, MIP-β and MCP-1 while IFN-γ, IL-10, IL-2 and IL-4 were unchanged (Zhao et al., 1997). To evaluate the effect of SALP encapsulation we conducted a similar study characterizing serum cytokine levels in ICR mice injected i.v. with 20 mg/kg of 16 mer c-myc PS ODN either in free form or encapsulated (SALP c-myc PS ODN) or with empty SALP vesicles. The serum cytokine levels which were characterized over a 24 h time course included those which influence Th1/Th2 responses (IL-12, IFN-γ, IL-2, IL-4, IL-6, IL-10), MCP-1 (a macrophage chemokine), and TNF-α (an inflamatory mediator). Of the Th1/Th2 associated cytokines, IL-12 and IFN-γ are strong promoters of Th1 responses while IL-4 and IL-10 promote Th2 responses. Injection of free c-myc PS ODN was found to induce a significant increase in IL-12 between 2 to 24 h after injection, with peak expression (a 20-fold increase compared to untreated mice) occurring at 4 h (Figure 28B). MCP-1 (Figure 28C) and IL-10 (Figure 29B) was weakly enhanced 2-3 fold while no significant differences were seen in IL-6 (Figure 28A), IFN-γ (Figure 1D), IL-2 (Figure 29A), IL-4 (Figure 29C), and TNF-α (Figure 29D) levels.

Encapsulating c-myc PS ODN in a liposome increased the mitogenicity of the ODN. Similar to free c-myc PS ODN, IL-12 levels were greatly enhanced >2h after injection of SALP c-myc PS ODN with peak expression occurring at 4 h (Figure 28B). The level of IL-12 induced with SALP c-myc PS ODN was 50-fold above baseline or 2.5 times more than with free c-myc PS ODN. Serum levels of IL-6 (1000-fold), MCP-1 (400-fold) and IFN-γ (20-fold) were also greatly enhanced with peak expression occurring at 4 h for IL-6 and MCP-1 and 8 h for IFN-γ (Figures 28A-D). TNF-α (Figure 29D) and IL-10 (Figure 29B) levels were slightly enhanced compared to untreated mice while IL-2 and IL-4 levels were unaffected. The effect of empty SALP was also investigated. An initial increase in IL-6 was seen 1h after injection which returned to baseline levels by 3 h (Figure 28A). MCP-1 and IL-12 levels were also slightly induced but alike IL-6, the effect was notably less compared to SALP c-myc PS ODN (Figures 28A-D). IFN-γ expression was unchanged. Thus, the induction of cytokine serum levels by SALP c-myc PS ODN was not due to an additive effect of the free ODN and lipid carrier.

*Effect of ODN backbone on serum cytokine induction*

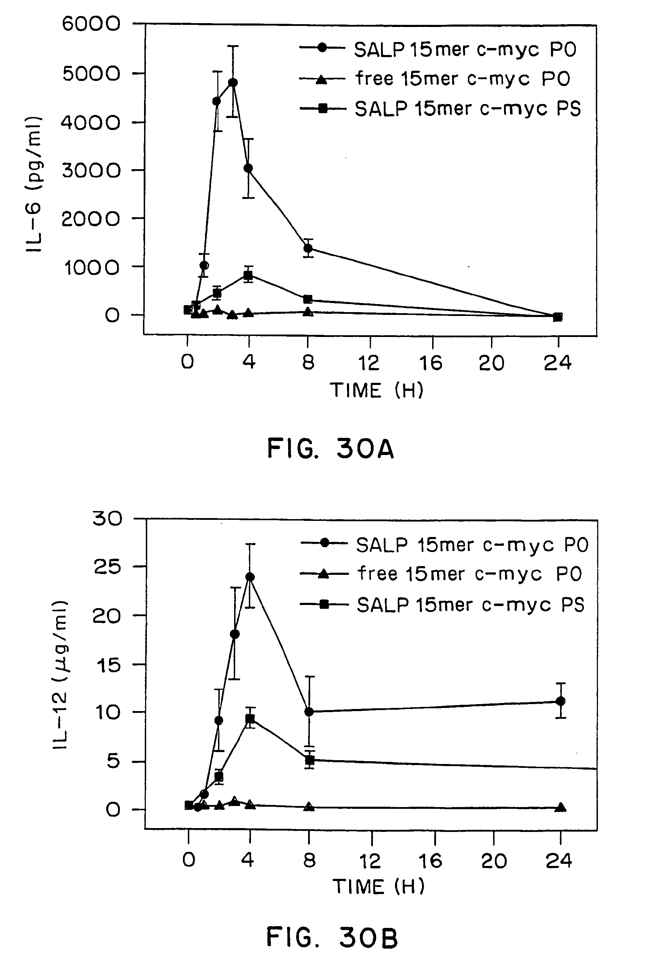

PO ODNs, being linear and single stranded, are rapidly degraded by serum nucleases (Fisher et al., 1993) and thus are not as immunogenic compared to the more stable phosphorothioate ODN in free form (Boggs et al., 1997). However, encapsulation of the ODN would protect it from degradation in the circulation. If the immune system have evolved to recognize bacterial DNA containing CpG motifs then an ODN with a normal phosphodiester backbone may be expected to be a more readily recognized and thus stimulatory compared to an ODN containing a chemically modified phosphorothioate backbone. Thus, it was of interest to compare the immunogenity of SALP formulations containing PS and PO ODN. Due to supply constraints, the 15 mer PO c-myc ODN was used in this experiment while the 16 mer c-myc ODN, which contains an extra thymidine at the 5' end, was used as the PS ODN. The known immunostimulatory sequence motif is the same for both ODNs, and the serum cytokine levels induced were shown to be the same in a control experiment comparing the serum cytokine levels induced by SALP containing the 16 mer PO or 16 mer c-myc PS ODN. No significant differences were found over a 7 day time course. As an additional experimental note, we have observed that between experiments there can be a ~2-fold difference in serum cytokine levels measured with similar samples. For example, although SALP c-myc PS ODN (16 mer) was used in the following experiment (Figures 30A-D) as well as the one shown in Figures 28A-D, $23 \pm 2$ µg/ml of IL-12 was detected at 4 h in the first experiment but only $10 \pm 1$ in the following study. The variability was not due to differences in SALP preparations (data not shown) but may arise from environmental conditions or genetic variability between different batches of ICR mice. Repeat experiments indicate, however, that the comparative differences observed between different sample types remain relatively unchanged.

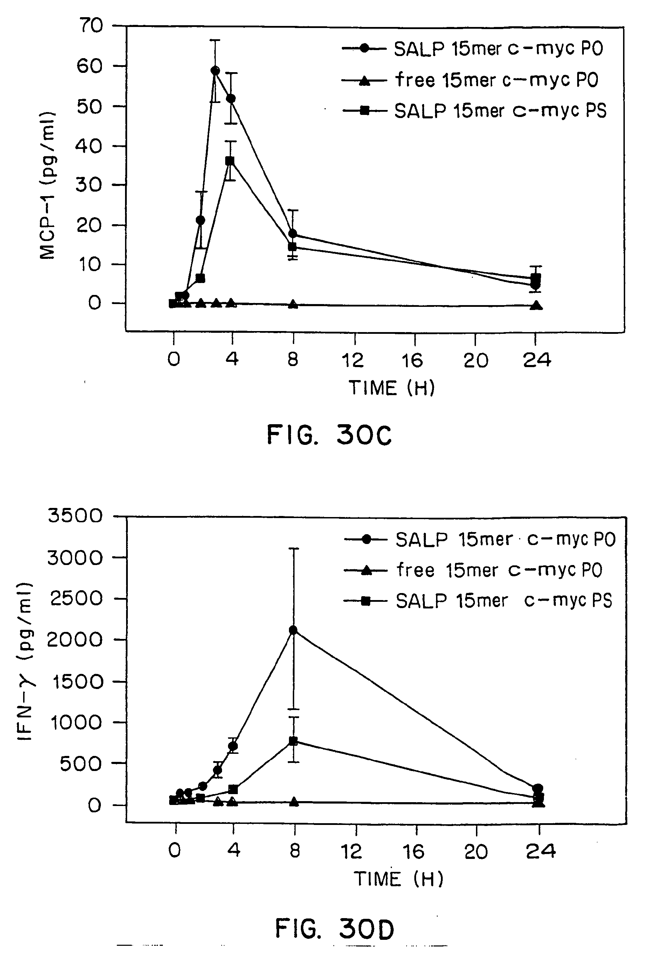

In the study shown in Figures 30A-D, ICR mice were injected with 20 mg/kg SALP c-myc PS ODN (16 mer), SALP c-myc PO ODN (15 mer) or free c-myc PO ODN (15 mer) and serum cytokine levels measured over a 8 day time course. In mice injected with SALP c-myc PS ODN, an increase in IL-6, IL-12, MCP-1 and IFN-γ serum levels were detected as before (Figure 28A-D), peaking at 4 h for IL-6 (Figure 30A), IL-12 (Figure 30B) and MCP-1 (Figure 30C) and 8 h for IFN-γ (Figure 30D). Mice injected with SALP c-myc PO ODN also display maximum serum cytokine induction at approximately 4 h or 8 h, however, the levels of cytokine expressed were greater. Serum cytokine levels of MCP-1 was increased 1.4 fold while a 2-4 fold increase were observed for IL-12, IFN-γ and IL-6 (Figure 3). No detectable change in serum cytokine levels was detected in mice injected with free c-myc PO ODN as expected due to the rapid degradation of phosphodiester ODN in the circulation.

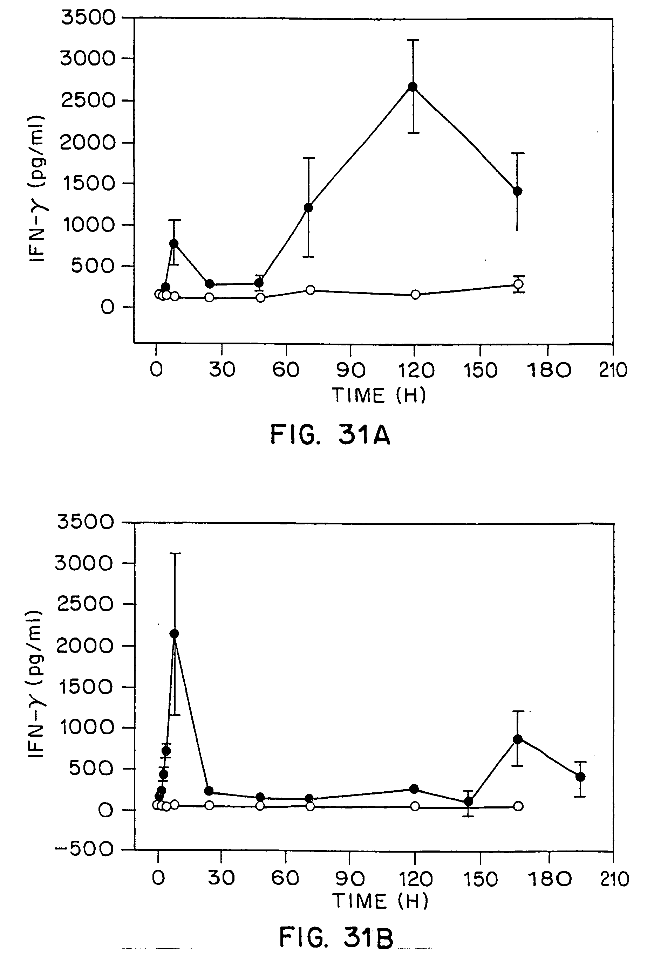

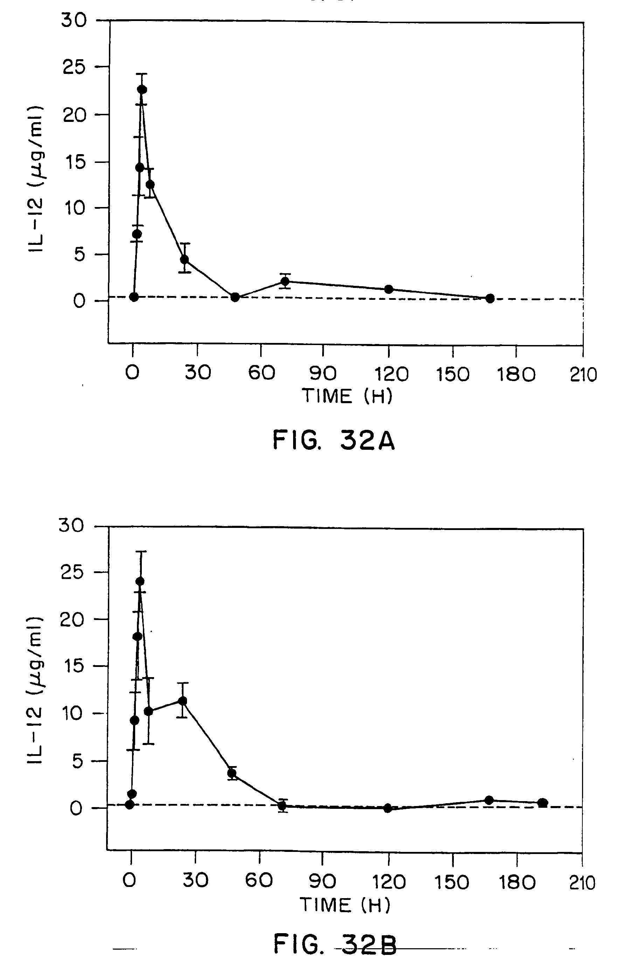

A second major difference between the effect of phosphorothioate and phosphodiester ODN-containing SALP was detected when we looked at IFN-γ levels beyond 24 h. In mice injected with SALP c-myc PS ODN a peak in IFN-γ levels occurred at 8 h, as indicated previously, but a second broad induction phase was seen between 2 and 7 days, peaking at approximately 5 days (Figure 31A). SALP c-myc PO ODN, which induced a higher level of IFN-γ at 8 h, also resulted in a second IFN-γ peak starting at approximately day 6 (Figure 31B). However, the amount expressed was significantly lower compared to that induced by SALP c-myc PS ODN. The difference in IFN-γ levels induced by the phosphorothioate and phosphodiester ODN-containing SALP's would suggest that the second IFN-γ phase is dependent on the presence of undegraded ODN. IFN-γ induced by polynucleotides have been identified as being secreted from NK cells *in vivo* in response to IL-12 released from macrophages (Chase et al., 1997). However, when serum IL-12 levels were analyzed a second IL-12 induction phase was not as evident. A very small increase in IL-12 was seen between 3 and 5 days (72 - 120 h) with SALP c-myc PS ODN (Figure 32A) and at 7 days (168 h) with SALP c-myc PO ODN (Figure 32B). The dotted line in Figure 5 represent IL-12 levels in HBS-injected mice. One explanation for the differences in relative IL-12 and IFN-γ levels at the two induction phases is that at the latter phase, there are more NK cells present (Bramson et al., submitted) and thus the effect of IL-12 would be amplified. Another possibly is that the release of IL-12, possibly arising from maturing dendritic cells, is localized. Immature dendritic cells, after taking up the SALP/ODN, would become activated and translocate to T-cell rich areas within draining lymph nodes, releasing IL-12 and stimulating T-cells and NK cells to produce IFN-γ. Free c-myc PS ODN also induced a second phase of IFN-γ expression but a higher (> 40 mg/kg) dose was needed to achieve a measurable (2 fold above baseline) difference while free c-myc PO ODN had no effect (data not shown). No significant increases in IL-6 and MCP-1 levels were detected beyond what could be associated with the tail end of the initial 4 h peak (Figures 28 and 30) over a course of 7 days. In addition, no change in IL-2, IL-4, IL-10 or TNF-α levels were detected.

*ODN sequence dependence*

The presence of ODN in the SALP formulation has been found to have an adaptive immunogenic effect in terms of inducing the recognition and clearance of vesicles containing polyethylene-conjugated lipids upon repeat injections (Semple et al., submitted). However, the response seen was independent of the ODN sequence as well as the whether a PS or PO ODN was used. Thus, we investigated the effect of the ODN sequence and backbone in the SALP with respect to the level of cytokine induced. Two ODN sequences were compared, the c-myc ODN and the non or weakly immunostimulatory ISIS 3082.

Mice were treated with 20 mg/kg of SALP c-myc PO ODN (15 mer), SALP ISIS 3082 PO ODN or free ISIS 3082 PO ODN and serum IL-6, IL-12, MCP-1 and IFN-γ levels measured over 7 days. The kinetics of serum cytokine induction were similar to what was previously observed with a 4 h peak expression occurring for IL-6, MCP-1 and IL-12 and 8 and 120 h for IFN-γ. The serum cytokine concentrations at these time points are tabulated in Table I along with results from the previous two studies (Figures 28 and 29). Serum levels of IL-6, MCP-1, IL-12 and IFN-γ were 2-10 fold lower in mice treated with SALP ISIS 3082 (PO) compared to SALP c-myc PO ODN. A similar effect is seen when the PS versions of the ODNs are compared. SALP c-myc PS ODN induced a 10 - 2000 fold higher expression of the above cytokines compared to SALP containing ISIS PS ODN.

*Effect of dose on cytokine induction*

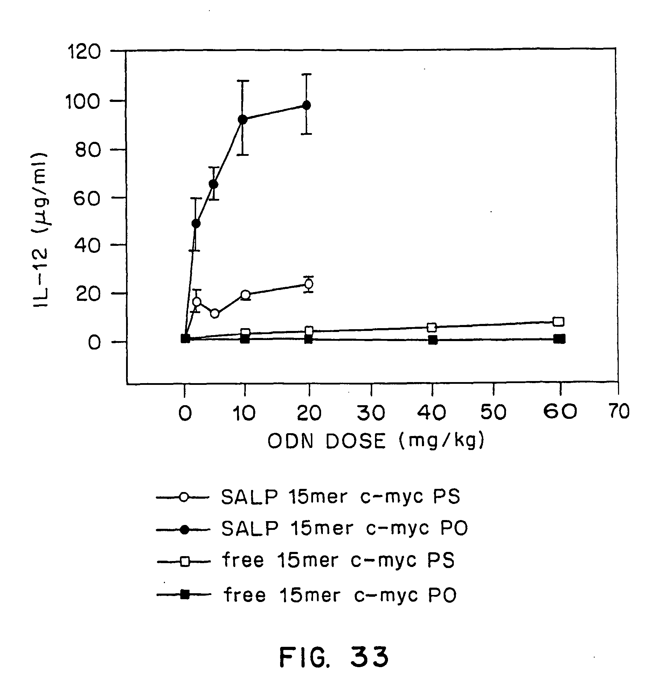

To better characterize the relative levels of cytokine induction conferred by SALP we performed a dose titration study with SALP c-myc PS ODN (15 mer), SALP c-myc P0 ODN (15 mer), free c-myc PS ODN (15 mer) and free c-myc PO ODN (15 mer). Mice were injected with 2 - 20 or 10 - 60 mg/kg of SALP or free ODN, respectively, and serum levels of IL-6, IL-12, MCP-1 and IFN-γ were measured. A typical dose titration shown for IL-12 indicate that even at 60 mg/kg of free c-myc PS ODN, IL-6, IL-12, MCP-1 and IFN-γ levels do not reached the same level compared to 5 mg/kg SALP c-myc PS ODN or SALP c-myc PO ODN (Figure 33).

The results of these experiments demonstrate that ODN encapsulated in a lipid carrier has increased immunogenicity. The increased immunogenicity of SALP compared to free ODN may be partly due to the enhanced stability of the ODN and increased biodistribution to macrophages. The former may explain the higher cytokine expression observed with encapsulated c-myc PO ODN compared to free PO ODN which would be rapidly degraded by serum nucleases. With the more nuclease resistent PS ODN, an increased ODN distribution to macrophages likely contributes to the enhanced immunogenicity of the SALP compared to free PS ODN. Encapsulated PO ODN was also more immune stimulatory than the corresponding SALP PS ODN, reflecting perhaps the pattern recognition receptors for CpG polynucleotides which would be expected to have stronger affinity for the natural PO backbone.

ISIS 3082 PO ODN, which does not contain CpG sequence motifs, also stimulated cytokine expression when administered in a SALP. It is unclear whether the effect is due to the ODN (non-CpG ODNs can stimulate both dendritic cells (Jakob et al., 1998) and B cells (Davis et al., 1998; Monteith et al., 1997) *in vitro* but much higher concentrations are required) or due to the lipid/ODN combination and not simply the ODN itself. Liposomes containing protein antigens tend to enhance Th1 -type responses, as evident by either the cytokines (IFN-γ) or antibody isotype (IgG2a) induced, even if the antigen alone has a Th2 bias response (Afrin & Ali, 1998; Krishnan et al., 2000; Sehra et al., 1998). At the cellular level, liposomal protein particles alters the intracellular trafficking pattern of both the lipid and protein in APCs such that antigens will also enter the MHC class I pathway (Rao & Alving, 2000). Both the Th1 biased response and association with MHC class I molecules are classical responses to intracellular pathogens such as viruses. A similar effect may occur with liposomes containing polynucleic acids which may be recognized as viral-like particles.

The induction of IL-12 by both free and encapsulated c-myc PS ODN indicate that a Th1-type response is induced. Further, IFN-γ, IL-6 and MCP-1 were greatly up-regulated when ODNs were administered in a SALP compared to free form (Table 2). Thus, SALP significantly enhanced an immune response but does not appear to change the type or kinetics of the cytokines that can be induced by ODNs (cf Zhao et al., 1997; Klinman et al., 1996). However, the relative expression of the cytokines induced is altered. For example, SALP increased IL-12 expression only 2-3 fold compared to the free c-myc PS ODN while IFN-γ expression was enhanced 1000 fold (Table 3). This is not simply due to an amplified effect of IL-12 on the downstream expression of IFN-γ as SALP ISIS 3082 induced a lower level of IL-12 at 4 h compared to free c-myc PS but stimulated >1000-fold expression of IFN-γ at 8 h.

Of the four cytokines which were greatly up-regulated in response to SALP, IL-12 and IFN-γ are known to be important or essential in the antitumor (Dow et al., 1999) effects of CpG ODNs and protection from infectious agents (Walker et al., 1999; Krieg et al.,1998; Schwartz et al., 1999; Zimmermann et al., 1998). Maximum induction of IFN-γ has been shown to be - 8 h for DNA/lipid particles (Dow et al., 1999; Whitmore et al., 1999), similar with the results in this study. SALP PO ODN induced a higher level of IFN-γ expression than SALP PS ODN at this early time point, however, when we extended the time course over 7 days we observed a second broader IFN-γ induction phase occurring at ~ 5 days (Figure 31). This second IFN-γ peak is greatest for SALP PS ODN but significantly smaller for P0 0DN-containing SALP, suggesting that the presence of an intact ODN is required. The absence of a correspondingly high serum IL-12 expression prior to this second IFN-γ peak suggests that the immune system has been altered or primed, possibly through expansion of NK cells (Bramson et al., 2000) or maturation of dendritic cells (Lipford, 1998). IFN-γ is involved in activation of macrophages and NK cells, inhibition of tumor angiogenisis, as well as modulating the adaptive immune response through induction of antibody isotype switching. IL-12, along with IFN-γ, promote Th1 responses. This cytokine exhibits anti-metastatic and anti-angiogenic properties and causes an intense infiltration of tumors by macrophages. Further, IL-12 is in clinical trials as an anticancer agent as it can inhibit growth and cause regression of more immunogenic tumors (Golab & Zagozdzon, 1999).

The expression of MCP-1, which can be produced by a variety of cells including endothelial and smooth muscle cells (Graves & Valente, 1991), highlights the involvement of monocytes and macrophages. In addition to its chemotactic effects, MCP-1 can also induce contact-dependent tumor cell lysis by up-regulation of adhesion molecules on macrophages (Shinohara et al., 2000). IL-6, which is released from B cells and macrophages in response to CpG ODNs *in vitro* (ref), is involved in stimulation of B cell differentiation and induction of acute phase proteins.

Unlike the difference in cytokine induction seen in this study, a dependence on the ODN sequence and backbone was not previously observed with SALP where SALP was found to induce immune recognition and subsequent clearance of PEG-lipid-containing vesicles. This could be due to a variety of reasons. It is possible that different anti-PEG antibody titers were produced by the different ODN-containing SALPs but was not detectable in the measured vesicle clearance rates. Alternatively, development of the adaptive response may not be as sensitive to the ODN compared to the initial innate response. For example, the priming signal needed to support an adaptive immune response may require just a threshold signal to support B-cell differentiation and proliferation whereas the ODN has more of a direct effect on macrophages.

The results reported herein indicate that encapsulation of ODNs in a liposomal vesicle such as SALP greatly increases the ODN's immunogenicity, complicating the outcome of any true antisense activity even with a relatively non-immunogenic ODN such as ISIS 3082. However, these results support the potential use of SALP in immune therapies. Free CpG ODNs are already being employed as adjuvants for protein based vaccines (ref) and as agents for protection from infection (ref) and in anticancer therapy (ref). The potential benefit seen with CpG ODNs is their ability to stimulate a Th1 bias adjuvant response which, as demonstrated in this study, can be significantly enhanced by SALP. The immune stimulatory effect of SALP could prove beneficial in activating tumor-associated macrophages to become tumoricidal, an approach that is currently being used with another immune modulator, muramyl dipeptide (Fidler et al., 1997; Worth et al., 1999). Further, immune stimulation may reduce the toxic side effects of anticancer drugs such as doxorubicin through induction of cytokines needed to prevent apoptosis of normal cells (Killion et al., 1996; Shinohara et al, 1998). From an adjuvant point of view, liposomes would co-localize the antigen and CpG ODN, an effect which is likely to enhance the humoral response. Studies are presently ongoing comparing the adjuvant qualities SALP with other common adjuvants such as monophosphoryl lipid A, aluminum salts, and Freund's adjuvant.

TABLE 2

| Comparison of serum cytokine levels induced by various ODN formulations | | | | | |
|---|---|---|---|---|---|
| | IL-6 (pg/ml) 4h | MCP-1 (μg/ml) 4h | IL-12 (μg/ml) 4h | IFN-γ (pg/ml) 8h | IFN-γ (pg/ml) 120h |
| free c-myc PS | 0 ± 5 | 2 ± 1 | 7 ± 2 | 80 ± 6 | 130 ± 10 |
| c-mycPO | 50 ± 10 | 0 ± 0 | 0 ± 1 | 101 ± 7 | 47 ± 5 |
| ISIS 3082 PS | 0 ± 2 | 0 ± 0 | 1 ± 0 | 43 ± 3 | 64 ± 2 |
| ISIS 3082 PO | 30 ± 10 | 2 ± 2 | 1 ± 1 | 110 ± 10 | 40 ± 1 |
| SALP c-myc PS | 900 ± 200 | 36 ± 5 | 12 ± 3 | 1700 ± 400 | 2200 ± 400 |
| c-myc PO | 3100 ± 600 | 51 ± 6 | 36 ± 5 | 5000 ± 2000 | 260 ± 40 |
| ISIS 3082 PS | 0 ± 3 | 0 ± 0 | 2 ± 1 | 60 ± 10 | 80 ± 10 |
| ISIS 3082 PO | 400 ± 100 | 7 ± 2 | 3 ± 2 | 2300 ± 500 | 34 ± 2 |
| HBS | 60 ± 80 | 0 ± 0 | 1 ± 1 | 100 ± 50 | 100 ± 50 |

The Examples provided illustrate certain embodiments of the invention. In a more general sense, however, the invention encompasses compositions and methods for providing therapeutic benefits to mammalian subjects (including humans) utilizing such compositions. The compositions of the invention are in the form of comprising a lipid membrane vesicle; and a nucleic acid fully encapsulated within said vesicle. Where stimulation of a response to a particular antigen is desired, the composition may also incorporate the antigen with the vesicle, for example via an association with the exterior surface of the vesicle.

Preferred compositions are those in which the nucleic acid comprises greater than 4% by weight of the composition.

The nucleic acid in the compositions of the invention may suitably be nucleic acids which are not complementary to the genome of the treated mammal, and which provide immunostimulation through a mechanism which does not depend on a complementary base-pairing interaction with nucleic acids of the mammal. Such nucleic acids will frequently contain an immunostimulating sequence, such as a CpG motif or an immune stimulating palindrome.

The nucleic acids used in the compositions of the invention may be nucleic acids which do not induce an immune response when administered in free form to a naïve mammal, or which suppress an immune response to an immune stimulating sequence of nucleotides when administered in free form to a naive mammal.

The nucleic acids may have exclusively phosphodiester internucleotide linkages or may be modified in which a way that they a plurality of phosphodiester internucleotide linkages in combination with modified intemucleotide linkages. The nucleic acids may also contain exclusively modified linkages, or a plurality of modified linkages. For example, the nucleic acid may contain exclusively phosphorothioate internucleotide linkages or a plurality of phosphorothioate internucleotide linkages.

The cationic lipid which is used in formulating the composition suitably is selected from DODAP, DODMA, DMDMA, DOTAP, DC-Chol, DDAB, DODAC, DMRIE, DOSPA and DOGS. In addition, the lipid formulation preferably includes an aggregation preventing compound, such as a PEG-lipid, a PAO-lipid or a ganglioside.

In addition to or instead of an antigen, the compositions of the invention can include a co-encapsulated cytotoxic agent such as doxorubicin. The lipid membrane vesicle fully encapsulates both the nucleic acid and the cytotoxic agent. Compositions of this type can be prepared by a method which is a further aspect if the invention. In this method, a therapeutic composition is prepared preparing lipid in ethanol; mixing lipid with oligonucleotide in aqueous buffer to form oligonucleotide loaded lipid vesicles; and exposing the oligonucleotide loaded lipid vesicles to a cytotoxic agent such that the cytotoxic agent actively accumulates in the interior space of said vesicle.

The compositions of the invention can be used in various methods to provide therapeutic benefits to mammals, including humans, through the use of a lipid-nucleic acid particle comprising a nucleic acid which is fully encapsulated in a lipid formulation comprising a cationic lipid in the manufacture of a medicament. Thus, the compositions or can be used to induce an immune response in a mammal, to activate B cells in a mammal or to treat neoplasia in a mammal having a neoplasia by a method comprising the steps of preparing a lipid-nucleic acid particle comprising a nucleic acid which is fully encapsulated in a lipid formulation, which lipid formulation comprises a cationic lipid; and administering the lipid-nucleic acid particle to the mammal.

When an antigen is included in the composition, the invention provides a method of inducing an immune response to the antigen comprising preparing a particle comprising a lipid membrane vesicle comprising a nucleic acid fully encapsulated within said vesicle and an antigen to which an immune response is desired associated with an external surface of said vesicle, and administering the particles to the mammalian subject to be treated.

A particular application of the invention is in the treatment of lymphoma. Thus, the invention provides a method of treating a lymphoma comprising administering to a subject/patient having a lymphoma an oligonucleotide containing a plurality of phosphodiester internucleotide linkages fully encapsulated in a lipid membrane vesicle, at a dose of 0.0075 - 75 mg/kg oligonucleotide. In one embodiment of this invention, the oligonucleotide is one which contains an immune stimulating sequence.

As demonstrated in the examples above, the utilization of a lipid carrier in the compositions in accordance with the invention allows a substantial reduction in the amount of oligonucleotide needed to achieve the desired stimulation of the immune system. In some cases, this is reflected in the fact that an oligonucleotide which had no apparent activity in the free form is useful for stimulating an immune response when provided in lipid-encapsulated form. In other cases, this is reflected in the fact that the amount of ODN necessary to achieve the same level of response with a lower dosage of ODN. Thus, in practicing a method employing an effective amount of oligonucleotide to stimulate an immune response in a mammal, the present invention provides the improvement comprising fully-encapsulating the oligonucleotide in a lipid vesicle and administering less than 20% of said effective amount of oligonucleotide to a mammalian subject, thereby obtaining a desired immune response in said mammalian subject.

**Claims**

1. Use of a composition comprising an oligodeoxynucleotide fully encapsulated in a lipid particle comprising a cationic lipid, a neutral lipid and an aggregation-preventing compound for the manufacture of a pharmaceutical preparation for stimulating cytokine secretion in a mammal.

2. Use according to claim 1, **characterized in that** the oligodeoxynucleotide is a non-sequence specific oligodeoxynucleotide.

3. Use according to claim 2, **characterized in that** the oligodeoxynucleotide includes at least one CpG motif.

4. Use according to any one of claims 1 to 3, **characterized in that** the oligodeoxynucleotide has no detectable immunostimulatory activity in the mammal in the absence of the lipid particle.

5. Use according to any one of claims 1 to 4, **characterized in that** the oligodeoxynucleotide consists of deoxynucleotide residues joined by phosphodiester linkages.

6. Use according to any one of claims 1 to 5, **characterized in that** the cationic lipid is selected from among DODAP, DODMA, DMDMA, DOTAP, DC-Chol, DDAB, DODAC; DMRIE; DOSPA and DOGS.

7. Use according to any one of claims 1 to 6, **characterized in that** the aggregation-preventing compound is an exchangeable steric barrier lipid.

8. Use according to claim 7, **characterized in that** the steric barrier lipid is a PEG-lipid or a PAO-lipid.

9. Use according to any one of claims 1 to 8, **characterized in that** the neutral lipid is DSPC or cholesterol.

**10.** Use according to any one of claims 1 to 9, **characterized in that** the oligodeoxynucleotide and the lipid components of the particle are present in a ratio by weight of from 0.001 to 0.45.

**11.** Use according to any one of claims 1 to 10, **characterized in that** the particles have a mean diameter of 50-200 nm.

**12.** Use according to any one of claims 1 to 11, **characterized in that** the composition further comprises an antigen molecule selected from among polypeptides comprising at least one epitope of a target antigen and nucleic acids encoding at least one epitope of the target antigen, and that the composition is used for preparing a medication for stimulating cytokine secretion and for an immune response to the target antigen.

**13.** Use according to claim 12, **characterized in that** the antigenic molecule is associated with the lipid particle.

**14.** Composition for stimulating cytokine secretion and/or an immune response in a mammal, **characterized in that** the composition comprises an oligodeoxynucleotide fully encapsulated in a lipid particle comprising a cationic lipid, a neutral lipid and an aggregation-preventing compound, wherein the oligodeoxynucleotide is a non-sequence specific oligonucleotide and/or wherein the composition further comprises an antigenic molecule selected from among polypeptides comprising at least one epitope of a target antigen of the mammal and nucleic acids encoding at least one epitope of the target antigen.

**15.** Composition according to claim 14, **characterized in that** the oligodeoxynucleotide comprises at least one CpG motive.

**16.** Composition according to claim 14 or 15, **characterized in that** the oligodeoxynucleotide consists of deoxynucleotide residues joined by phosphodiester linkages.

**17.** Composition according to any one of claims 14 to 16, **characterized in that** the cationic lipid is selected from among DODAP, DODMA, DMDMA, DOTAP, DC-Chol, DDAB, DODAC; DMRIE; DOSPA and DOGS.

**18.** Composition according to any one of claims 14 to 17, **characterized in that** the aggregation-preventing compound is an exchangeable steric barrier lipid.

**19.** Composition according to claim 18, **characterized in that** the exchangeable steric barrier lipid is a PEG-lipid or a PAO-lipid.

**20.** Composition according to any one of claims 14 to 19, **characterized in that** the neutral lipid is DSPC and/or cholesterol.

**21.** Composition according to any of claims 14 to 20, **characterized in that** the oligodeoxynucleotide and the lipid components of the particle are present in a ratio by weight of from 0.001 to 0.45.

**22.** Composition according to any one of claims 14 to 21, **characterized in that** the particles have a mean diameter of 50-200 nm.

**23.** Composition according to any one of claims 14 to 22, **characterized in that** the antigenic molecule is associated with the lipid particle.

**24.** Composition according to any one of claims 14 to 23, **characterized in that** the oligodeoxynucleotide has no detectable immunostimulatory activity in the mammal in the absence of the lipid particle.

**Patentansprüche**

**1.** Verwendung einer Zusammensetzung, die ein Oligodeoxynucleotid umfasst, das vollständig in einem Lipidpartikel verkapselt ist, welches ein kationisches Lipid, ein neutrales Lipid und eine Verbindung umfasst, welche eine Aggregation verhindert, zur Herstellung einer pharmazeutischen Zubereitung zur Stimulation einer Cytokin Sekretion in einem Säugetier.

**2.** Verwendung gemäß Anspruch 1, **dadurch gekennzeichnet, dass** das Oligodexynucleotid ein nicht-sequenzspezifisches Oligodeoxynucleotid ist.

3. Verwendung gemäß Anspruch 2, **dadurch gekennzeichnet, dass** das Oligodeoxynucleotid zumindest ein CpG Motiv umfasst.

4. Verwendung gemäß einem der Ansprüche 1 bis 3, **dadurch gekennzeichnet, dass** das Oligodeoxynucleotid keine detektierbare immunstimulatorische Aktivität in dem Säugetier in Abwesenheit des Lipidpartikels hat.

5. Verwendung gemäß einem der Ansprüche 1 bis 4, **dadurch gekennzeichnet, dass** das Oligodeoxinucleotid aus Deoxinucleotidresten besteht, die über Phosphodiester-Brücken verbunden sind.

6. Verbindungen gemäß einem der Ansprüche 1 bis 5, **dadurch gekennzeichnet, dass** das kationische Lipid ausgewählt ist aus DODAP, DODMA, DMDMA, DOTAP, DC-Chol, DDAB, DODAC, DMRIE, DOSPA und DOGS.

7. Verwendung gemäß einem der Ansprüche 1 bis 6, **dadurch gekennzeichnet, dass** die Verbindung, welche die Aggregation verhindert, ein Lipid ist, das eine austauschbare sterische Barriere bildet.

8. Verwendung gemäß Anspruch 7, **dadurch gekennzeichnet, dass** das Lipid, das die sterische Barriere bildet, ein PEG-Lipid oder ein PAO-Lipid ist.

9. Verwendung gemäß einem der Ansprüche 1 bis 8, **dadurch gekennzeichnet, dass** das neutrale Lipid DSPC oder Cholesterol ist.

10. Verwendung gemäß einem der Ansprüche 1 bis 9, **dadurch gekennzeichnet, dass** das Oligodeoxynucleotid und die Lipidkomponenten des Partikels in einem Gewichtsverhältnis von 0,001 bis 0,45 vorliegen.

11. Verwendung gemäß einem der Ansprüche 1 bis 10, **dadurch gekennzeichnet, dass** die Lipidpartikel einen mittleren Durchmesser von 50 bis 200 Nanometer haben.

12. Verwendung gemäß einem der Ansprüche 1 bis 11, **dadurch gekennzeichnet, dass** die Zusammensetzung weiter ein Antigenmolekül umfasst, das ausgewählt ist aus Polypeptiden umfassend zumindest ein Epitop eines Targetantigens und Nucleinsäuren, die zumindest ein Epitop des Targetantigens kodieren, und dass die Zusammensetzung zur Herstellung einer Medizin zur Stimulierung einer Cytokin Sekretion und zur Immunantwort auf das Targetantigen verwendet wird.

13. Verwendung gemäß Anspruch 12, **dadurch gekennzeichnet, dass** das Antigen-Molekül mit dem Lipidpartikel assoziiert ist.

14. Zusammensetzung zur Stimulierung einer Cytokin Sekretion und/ oder einer Imunantwort in einem Säugetier, **dadurch gekennzeichnet, dass** die Zusammensetzung ein Oligodeoxynucleotid umfasst, das vollständig in einem Lipidpartikel verkapselt ist, wobei das Lipidpartikel ein kationisches Lipid, ein neutrales Lipid und eine Verbindung umfasst, die Aggregation verhindert, wobei das Oligodeoxynucletid ein nicht-sequenzspezifisches Oligonucleotid ist und/oder wobei die Zusammensetzung weiter ein Antigen-Molekül umfasst, das ausgewählt ist aus Polypeptiden, die zumindest ein Epitop eines Targetantigens des Säugetiers und Nucleinsäuren, die zumindest ein Epitop des Targetantigens kodieren, umfassen.

15. Zusammensetzung gemäß Anspruch 14, **dadurch gekennzeichnet, dass** das Oligodeoxynucleotid zumindest ein CpG-Motiv umfasst.

16. Zusammensetzung gemäß Anspruch 14 oder 15, **dadurch gekennzeichnet, dass** das Oligodeoxynucleotid aus Deoxynucleo-tidresten besteht, die durch Phosphodiester- Brücken verbunden sind.

17. Zusammensetzung gemäß einem der Ansprüche 14 bis 16 , **dadurch gekennzeichnet, dass** das kationische Lipid ausgewählt ist aus DODAP, DODMA, DMDMA, DOTAP, DC-Chol, DDAB, DODAC, DMRIE, DOSPA und DOGS.

18. Zusammensetzung gemäß einem der Ansprüche 14 bis 17, **dadurch gekennzeichnet, dass** die Verbindung, welche die Aggregation verhindert, ein Lipid ist, das eine austauschbare sterische Barriere bildet.

**19.** Zusammensetzung gemäß Anspruch 18, **dadurch gekennzeichnet, dass** das Lipid, das eine austauschbare sterische Barriere bildet, ein PEG- Lipid oder ein PAO- Lipid ist.

**20.** Zusammensetzung gemäß einem der Ansprüche 14 bis 19, **dadurch gekennzeichnet, dass** das neutrale Lipid DSPC und/oder Cholesterol ist.

**21.** Zusammensetzung gemäß einem der Ansprüche 14 bis 20, **dadurch gekennzeichnet, dass** das Oligodeoxynucleotid und die Lipidkomponenten des Partikels in einem Gewichtsverhältnis von 0,001 bis 0,45 vorhanden sind.

**22.** Zusammensetzung gemäß einem der Ansprüche 14 bis 21, **dadurch gekennzeichnet, dass** die Partikel einen durchschnittlichen Durchmesser von 50 bis 200 Nanometern haben.

**23.** Zusammensetzung gemäß einem der Ansprüche 14 bis 22, **dadurch gekennzeichnet, dass** das Antigen-Molekül mit dem Lipidpartikel assoziiert ist.

**24.** Zusammensetzung gemäß einem der Ansprüche 14 bis 23, **dadurch gekennzeichnet, dass** das Oligodeoxynucleotid keine detektierbare immunstimulatorische Aktivität in dem Säugetier in Abwesenheit des Lipidpartikels hat.

**Revendications**

**1.** Utilisation d'une composition comprenant un oligodésoxynucléotide complètement encapsulé dans une particule lipidique comprenant un lipide cationique, un lipide neutre et un composé antiagrégant pour la fabrication d'une composition pharmaceutique destinée à stimuler la sécrétion de cytokines chez un mammifère.

**2.** Utilisation selon la revendication 1, **caractérisée en ce que** l'oligodésoxynucléotide est un oligodésoxynucléotide non spécifique d'une séquence.

**3.** Utilisation selon la revendication 2, **caractérisée en ce que** l'oligodésoxynucléotide comprend au moins un motif CpG.

**4.** Utilisation selon l'une quelconque des revendications 1 à 3, **caractérisée en ce que** l'oligodésoxynucléotide ne présente pas d'activité immunostimulante détectable chez le mammifère en l'absence de la particule lipidique.

**5.** Utilisation selon l'une quelconque des revendications 1 à 4, **caractérisée en ce que** l'oligodésoxynucléotide consiste en des résidus de désoxynucléotide reliés par des liaisons phosphodiester.

**6.** Utilisation selon l'une quelconque des revendications 1 à 5, **caractérisée en ce que** le lipide cationique est choisi parmi DODAP, DODMA, DMDMA, DOTAP, DC-Chol, DDAB, DODAC ; DMRIE ; DOSPA et DOGS.

**7.** Utilisation selon l'une quelconque des revendications 1 à 6, **caractérisée en ce que** le composé antiagrégant est un lipide à barrière stérique échangeable.

**8.** Utilisation selon la revendication 7, **caractérisée en ce que** le lipide à barrière stérique est un PEG ou un PAO.

**9.** Utilisation selon l'une quelconque des revendications 1 à 8, **caractérisée en ce que** le lipide neutre est le DSPC ou le cholestérol.

**10.** Utilisation selon l'une quelconque des revendications 1 à 9, **caractérisée en ce que** l'oligodésoxynucléotide et les composants lipidiques de la particule sont présents dans un rapport pondéral de 0,001 à 0,45.

**11.** Utilisation selon l'une quelconque des revendications 1 à 10, **caractérisée en ce que** les particules ont un diamètre moyen de 50-200 nm.

**12.** Utilisation selon l'une quelconque des revendications 1 à 11, **caractérisée en ce que** la composition comprend, en outre, un antigène choisi parmi les polypeptides comprenant au moins un épitope d'un antigène cible et des acides nucléiques codant au moins un épitope de l'antigène cible, et **en ce que** la composition est utilisée pour préparer un médicament destiné à stimuler la sécrétion de cytokine et pour une réponse immune à l'antigène cible.

**13.** Utilisation selon la revendication 12, **caractérisée en ce que** la molécule antigène est associée à la particule lipidique.

**14.** Composition pour stimuler la sécrétion de cytokines et/ou une réponse immune chez un mammifère, **caractérisée en ce que** la composition comprend un oligodésoxynucléotide complètement encapsulé dans une particule lipidique comprenant un lipide cationique, un lipide neutre et une composition antiagrégante, dans laquelle l'oligodésoxynucléotide est un oligonucléotide non spécifique d'une séquence et/ou dans lequel la composition comprend, en outre, un antigène choisi parmi les polypeptides comprenant au moins un épitope d'un antigène cible du mammifère et des acides nucléiques codant au moins un épitope de l'antigène cible.

**15.** Composition selon la revendication 14, **caractérisée en ce que** l'oligodésoxynucléotide comprend au moins un motif CpG.

**16.** Composition selon la revendication 14 ou 15, **caractérisée en ce que** l'oligodésoxynucléotide consiste en des résidus de désoxynucléotide reliés par des liaisons phosphodiesters.

**17.** Composition selon l'une quelconque des revendications 14 à 16, **caractérisée en ce que** le lipide cationique est choisi parmi DODAP, DODMA, DMDMA, DOTAP, DC-Chol, DDAB, DODAC ; DMRIE ; DOSPA et DOGS.

**18.** Composition selon l'une quelconque des revendications 14 à 17, **caractérisée en ce que** le composé antiagrégant est un lipide à barrière stérique échangeable.

**19.** Composition selon la revendication 18, **caractérisée en ce que** le lipide à barrière stérique échangeable est un PEG ou un PAO.

**20.** Composition selon l'une quelconque des revendications 14 à 19, **caractérisée en ce que** le lipide neutre est le DSPC et/ou le cholestérol.

**21.** Composition selon l'une quelconque des revendications 14 à 20, **caractérisée en ce que** l'oligodésoxynucléotide et les composants lipidiques de la particule sont présents dans un rapport pondéral de 0,001 à 0,45.

**22.** Composition selon l'une quelconque des revendications 14 à 21, **caractérisée en ce que** les particules ont un diamètre moyen de 50-200 nm.

**23.** Composition selon l'une quelconque des revendications 14 à 22, **caractérisée en ce que** l'antigène est associé à la particule lipidique.

**24.** Composition selon l'une quelconque des revendications 14 à 23, **caractérisée en ce que** l'oligodésoxynucléotide ne présente pas d'activité immunostimulante détectable chez le mammifère en absence de la particule lipidique.

FIG. 1A

FIG. 1B

FIG. 1C

FIG. 2A

FIG. 2B

FIG. 3

FIG. 4

FIG. 5A

FIG. 5B

FIG. 6

FIG. 7A

FIG. 7B

FIG. 8A

FIG. 8B

FIG. 9

FIG. 10

**% SURVIVAL** vs **TIME (DAYS)**

- —●— HBS
- —○— INX-6295 (2 mg/kg)
- —▼— INX-6295 (5 mg/kg)
- —▽— AS4200/INX-6295 (2 mg/kg)
- —■— AS4200/INX-6295 (5 mg/kg)
- —□— AS4204/INX-6295 (2 mg/kg)
- —◆— AS4204/INX-6295 (5 mg/kg)
- —◇— INX-6300 (5 mg/kg)
- —▲— AS4200/INX-6300 (5 mg/kg)
- —△— AS4204/INX-6300 (5 mg/kg)

## FIG. 11A

FIG. 11B

FIG. 12

STIMULATION INDEX
(RELATIVE TO MEDIA CONTROL)

FIG. 13

FIG. 14

FIG. 15

FIG. 16

FIG. 17

FIG. 18A

FIG. 18B

FIG. 19A

FIG. 19B

FIG. 20

FIG. 21

FIG. 22A

FIG. 22B

FIG. 23A

FIG. 23B

FIG. 23C

FIG. 24A

FIG. 24B

FIG. 24C

FIG. 25

FIG. 26A

FIG. 26B

FIG. 26C

FIG. 27

FIG. 28A

FIG. 28B

FIG. 28C

FIG. 28D

FIG. 29A

FIG. 29B

FIG. 29C

FIG. 29D

FIG. 30A

FIG. 30B

FIG. 30C

FIG. 30D

FIG. 31A

FIG. 31B

FIG. 32A

FIG. 32B

FIG. 33

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9840100 A, Davis, HL
- WO 9851278 A
- WO 9611266 A
- WO 9602555 A
- WO 9818810 A
- US 5663153 A
- US 5723335 A
- US 4320121 A, Sears
- US 5820873 A, Choi
- US 5885613 A, Holland
- US 218988 A
- US 09094540 B
- US 09218988 B
- US 835281 A
- US 60083294 B
- US 5705385 A
- US 08660025 B
- US 09140476 B
- US 08484282 B
- US 08856374 B
- US 09078954 B
- US 09078955 B
- US 60143978 B
- WO 9640964 A
- US 856374 A
- WO 60176406 A
- WO 60151211 A

**Non-patent literature cited in the description**

- **YAMAMOTO S. et al.** *J. Immunol.,* 1992, vol. 148, 4072-4076
- **KREIG, A.M. et al.** *Nature,* 1995, vol. 374, 546-549
- **KRIEG, AM.** *J Gene Med,* 1999, vol. 1, 56-63
- **KLIBANOV et al.** *FEBS Letters,* 1990, vol. 268 (1), 235-237
- **WHEELER, J. J. ; PALMER, L. ; OSSANLOU, M. ; MACLACHLAN, I. ; GRAHAM, R. W. ; ZHANG, Y. P. ; HOPE, M. J. ; SCHERRER, P. ; CULLIS, P. R.** *Gene Ther.,* 1999, vol. 6, 271-281
- **MORTIMER I ; TAM P ; MACLACHLAN I ; GRAHAM RW ; SARAVOLAC EG ; JOSHI PB.** Cationic lipid mediated transfection of cells in culture requires mitotic activity. *Gene Ther.,* 1999, vol. 6, 403-411
- **KLUIN-NELEMANS HC et al.** *Leukemia,* 1991, vol. 5 (3), 221-224
- **LEONETTI, C. et al.** *J. Nat. Canc. Inst.,* 1996, vol. 88 (7), 419-429